(19) Europäisches Patentamt
European Patent Office
Office européen des brevets

(11) **EP 3 168 251 B1**

(12) **EUROPÄISCHE PATENTSCHRIFT**

(45) Veröffentlichungstag und Bekanntmachung des Hinweises auf die Patenterteilung:
**06.02.2019 Patentblatt 2019/06**

(51) Int Cl.:
*C08G 63/672* *(2006.01)*          *C08G 63/668* *(2006.01)*
*A61Q 1/14* *(2006.01)*          *A61Q 5/02* *(2006.01)*
*A61Q 19/10* *(2006.01)*

(21) Anmeldenummer: **15194652.2**

(22) Anmeldetag: **16.11.2015**

(54) **VERNETZTE POLYGLYCERINESTER**

CROSSLINKED POLYGLYCEROL ESTERS

ESTERS DE POLYGLYCERINE RETICULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.05.2017 Patentblatt 2017/20**

(73) Patentinhaber: **Evonik Degussa GmbH**
**45128 Essen (DE)**

(72) Erfinder:
• **SCHUCH, Dominik**
**42781 Haan (DE)**

• **HARTUNG, Christian**
**45133 Essen (DE)**
• **BERKELS, Wolfgang**
**46238 Bottrop (DE)**

(56) Entgegenhaltungen:
**EP-A2- 1 683 781**

Bemerkungen:
Die Akte enthält technische Angaben, die nach dem Eingang der Anmeldung eingereicht wurden und die nicht in dieser Patentschrift enthalten sind.

EP 3 168 251 B1

**Beschreibung**

Gebiet der Erfindung

Gegenstand der Erfindung sind Polyglycerinpartialester auf Basis von Mono- und Dicarbonsäuren und deren Verwendung als Solubilisatoren, insbesondere in der Kosmetik, zum Beispiel für Parfumöle und essentielle Öle in wässrigen Systemen.

Stand der Technik

Die WO2012007754 offenbart Polyglycerinpartialester, die durch Umsetzung von Polyglycerin mit 3 bis 20 Glycerin-Einheiten mit einer Dicarbonsäure oder einem cyclischen Anhydrid einer derartigen Dicarbonsäure mit 4 bis 22 Kohlenstoffatomen und einer Monocarbonsäure mit 4 bis 24 Kohlenstoffatomen in einem molaren Verhältnis von 1,5: 1,0: 0,1 bis 3,0: 1,0: 3,0 erhalten werden, sowie die Verwendung dieser Polyglycerinpartialester als Emulgator, Lösungsvermittler und / oder Verdickungsmittel in Körperpflege- und/oder Heimpflege-Formulierungen.

Die EP0835862 beschreibt Polyglycerinpartialester von gesättigten oder ungesättigten, linearen oder verzweigten Fettsäuren und mehrfunktionellen Carbonsäuren, erhältlich durch Veresterung eines Polyglyceringemisches mit gesättigten oder ungesättigten, linearen oder verzweigten Fettsäuren mit 12 bis 22 C-Atomen und mehrfunktionellen Carbonsäuren mit 4 bis 54 C-Atomen und einer mittleren Funktionalität von 2 bis 2,4, wobei der Veresterungsgrad der Polyglycerinmischung zwischen 30 und 75 % liegt.

Die EP1683781 beschreibt Polyglycerinpartialester von Polyricinolsäure und mehrfunktionellen Carbonsäuren, erhältlich durch Veresterung a) eines Polyglyceringemisches mit b) mindestens einer bestimmten Polyricinolsäure und gegebenenfalls b1) Polyhydroxystearinsäure und c) mindestens einer Di- und/oder Tricarbonsäure und d) mindestens einer Fettsäure nach an sich bekannten Verfahren. Das Polyglycerin weist bevorzugt einen mittleren Kondensationsgrad n von 1 bis 11, bevorzugt 2 bis 6, auf.

Die EP1500427 beschreibt Polyglycerinpartialester von Polyhydroxystearinsäure und mehrfunktionellen Carbonsäuren, erhältlich durch Veresterung eines Polyglyceringemisches mit Polyhydroxystearinsäure und Di- und/oder Tricarbonsäuren und gegebenenfalls/oder mit Dimerfettsäuren und Fettsäuren mit 6 bis 22 C-Atomen.

Ein Nachteil der im Stand der Technik beschriebenen Polyglycerinpartialester ist es, dass diese nicht in der Lage sind, relativ polare Öle mit geringeren Überschüssen an Polyglycerinpartialester klar in Wasser oder wässrigen Formulierungen zu solubilisieren. Mit relativ polaren Ölen sind beispielsweise essentielle Öle und Parfumöle gemeint, beispielsweise Rosmarin- oder Zitronenöl. Polyglycerinester mit hohen Anteilen an Dicarbonsäure neigen dazu, dass Formulierungen zum Solubilisieren von Ölen nicht klar sind und eine Opaleszenz aufweisen.

Ein weiterer Nachteil der im Stand der Technik beschriebenen Verbindungen ist es, dass diese nicht in der Lage sind, Parfumöle mit geringeren Überschüssen an Polyglycerinpartialester klar in aluminiumchlorohydrathaltigen oder anderen stark elektrolythaltigen Formulierungen zu solubilisieren.

Ein weiterer Nachteil der im Stand der Technik beschriebenen Verbindungen ist es, dass diese oft, vor allem aufgrund hoher Viskositäten der Produkte, relativ schwer zu formulieren sind, oder dass während der Herstellung der Formulierung schwierige oder langwierige Formulierungsarbeit notwendig ist.

Aufgabe der Erfindung war es, Polyglycerinpartialester bereitzustellen, mit denen sich essentielle Öle und Parfumöle unter Verwendung geringer Mengen an Polyglycerinpartialester klar und einfach in Wasser, in wässrigen Formulierungen, in aluminiumchlorohydrathaltigen oder anderen stark elektrolythaltigen Formulierungen solubilisieren lassen.

Beschreibung der Erfindung

Überraschenderweise wurde gefunden, dass die im Folgenden beschriebenen Polyglycerinpartialester die der Erfindung gestellte Aufgabe zu lösen vermögen.

Gegenstand der vorliegenden Erfindung ist daher ein Polyglycerinpartialester erhältlich durch Veresterung eines Polyglycerins mit einer Carbonsäuremischung umfassend:

   a) mindestens eine kurzkettige Dicarbonsäure aufweisend 2 bis 12, bevorzugt 3 bis 8, besonders bevorzugt 4, Kohlenstoffatome, und
   b) mindestens eine gesättigte Fettsäure aufweisend 6 bis 14, bevorzugt 8 bis 10, Kohlenstoffatome,

dadurch gekennzeichnet, dass das molare Verhältnis von Polyglycerin zu Dicarbonsäure zu gesättigter Fettsäure von 3,1 : 1,0 : 0,5 bis 14 : 1,0 : 6,0, bevorzugt von 3,3 : 1,0 : 0,7 bis 7,0 : 1,0 : 3,0, beträgt.

Ein weiterer Gegenstand der Erfindung ist die Verwendung entsprechender Polyglycerinpartialester als Solubilisator.

Ein Vorteil der vorliegenden Erfindung ist, dass die hier beschriebenen Polyglycerinpartialester in der Lage sind, hydrophobe, öllösliche Substanzen, wie z.B. essentielle Öle, klar in Wasser oder einer kosmetischen Formulierung zu solubilisieren.

Ein weiterer Vorteil ist, dass die hier beschriebenen Polyglycerinpartialester im Gegensatz zu polyethoxylierten Triglyceriden (z.B. PEG-40 Hydrogenated Castor Oil) aus ausschließlich nachwachsenden Rohstoffen hergestellt werden können.

Noch ein Vorteil der vorliegenden Erfindung ist, dass man Formulierungen bereitstellen kann, die polyglycoletherfrei sind.

Ein weiterer Vorteil ist, dass die hier beschriebenen Polyglycerinpartialester im Gegensatz zu polyethoxylierten Triglyceriden flüssig und somit gut verarbeitbar sind. Auch im Vergleich zu Polyglycerinestern des Standes der Technik sind die erfindungsgemäßen Produkte verhältnismäßig leicht zu verarbeiten. Aufgrund der vergleichsweise niedrigen Produktviskositäten kann die Fließfähigkeit durch geringe Mengen zugesetzter Solventien, wie beispielsweise Wasser oder 1,2- sowie 1,3-Propandiol oder Glycerin, weiter verbessert werden, um eine noch bessere Verarbeitbarkeit zu gewährleisten.

Ein weiterer Vorteil gegenüber den polyethoxylierten Triglyceriden und teilweise auch den Polyglycerinestern des Stands der Technik ist, dass die hier beschriebenen Polyglycerinester zu augenscheinlich besonders klaren Dispersionen des Öls im Wasser führen und auch bei Lagerung keine Eintrübung erfolgt.

Ein weiterer Vorteil der hier beschriebenen Polyglycerinpartialester ist, dass diese ein angenehmes Hautgefühl in kosmetischen Formulierungen erzeugen können.

Noch ein Vorteil der hier beschriebenen Polyglycerinpartialester ist, dass diese in Wasser unter Rühren nur eine sehr geringe Schaumbildung zeigen.

Ein weiterer Vorteil ist, dass die hier beschrieben Polyglycerinpartialester eine nur sehr geringe Wirkung auf Anschäumbarkeit und Schaummenge in Tensidformulierungen zeigen, dabei aber die Schaumcremigkeit verbessern können.

Noch ein Vorteil ist, dass die hier beschriebenen Polyglycerinpartialester in Tensidformulierungen zur Abmilderung der Reizwirkung auf der Haut führen können.

Noch ein Vorteil ist, dass die hier beschriebenen Polyglycerinpartialester in kosmetischen Formulierungen als Feuchtigkeitsspender (Humectant) fungieren können.

Ein weiterer Vorteil der hier beschriebenen Polyglycerinpartialester ist, dass diese in Emulsionen eine stabilisierende Wirkung aufweisen können.

Noch ein Vorteil der erfindungsgemäßen Produkte ist, dass diese besonders gute Ergebnisse in Make-up-Entfernern zeigen.

Ein weiterer Vorteil der erfindungsgemäßen Produkte ist, dass diese einen relativ geringen Effekt auf die Viskosität von Tensidformulierungen aufweisen.

Ein weiterer Vorteil der erfindungsgemäßen Produkte ist, dass diese relativ stabil gegenüber Oxidation sind und stabil bezüglich Farbe, Geruch und Aussehen sind.

Noch ein weiterer Vorteil der erfindungsgemäßen Produkte ist, dass diese gegenüber Elektrolyten (wie Natriumchlorid, Aluminiumsalze oder kationische Tenside) in Formulierungen besonders kompatibel sind.

Die erfindungsgemäßen Polyglycerinpartialester stellen Mischungen von unterschiedlichen Substanzen dar; daher ist dem Fachmann klar, dass die angegebenen Zahlenwerte Mittelwerte über die Mischung darstellen.

Unter dem Begriff "Polyglycerin" im Sinne der vorliegenden Erfindung ist ein Polyglycerin zu verstehen, welches auch Glycerin enthalten kann. Somit ist zur Berechnung von Mengen, Massen und dergleichen gegebenenfalls ein Glycerinanteil mit zu berücksichtigen. Das Polyglycerin stellt aufgrund seiner polymeren Eigenschaft eine statistische Mischung verschiedener Verbindungen dar. Polyglycerin kann ausgebildete Etherbindungen zwischen zwei primären, einer primären und einer sekundären sowie zwei sekundären Positionen der Glycerinmonomere aufweisen. Aus diesem Grund besteht das Polyglycerin-Grundgerüst üblicherweise nicht ausschließlich aus linear verknüpften Glycerin-Einheiten, sondern kann auch Verzweigungen und Cyclen enthalten. Für Details siehe z.B. *"Original synthesis of linear, branched and cyclic oligoglycerol Standards"*, Cassel et al., J. Org. Chem. 2001, 875-896.

Analoges gilt für den Begriff "Polyglycerinpartialester" im Zusammenhang mit der vorliegenden Erfindung. Alle angegebenen Prozent (%) sind, wenn nicht anders angegeben, Massenprozent.

Erfindungsgemäß bevorzugte Polyglycerinpartialester sind dadurch gekennzeichnet, dass das eingesetzte Polyglycerin einen mittleren Kondensationsgrad $N$ von 1,5 bis 9, bevorzugt 2 bis 7, besonders bevorzugt 2,5 bis 5 aufweist.

Der mittlere Polymerisationsgrad des Polyglycerins $N$ wird über dessen Hydroxylzahl (OHV, in mg KOH/g) gemäß folgender Formel berechnet:

$$N = \frac{(112200 - 18 \cdot OHV)}{(74 \cdot OHV - 56100)}$$

Geeignete Bestimmungsmethoden zur Ermittlung der Hydroxylzahl sind insbesondere solche gemäß DGF C-V 17 a (53), Ph. Eur. 2.5.3 Method A und DIN 53240.

Somit ist erfindungsgemäß bevorzugter Polyglycerinpartialester dadurch gekennzeichnet, dass das eingesetzte Polyglycerin eine Hydroxylzahl von 1500 bis 900, bevorzugt 1350 bis 940, besonders bevorzugt 1245 bis 1010 mg KOH / g aufweist.

Das eingesetzte Polyglycerin kann durch verschiedene konventionelle Methoden wie beispielsweise Polymerisation von Glycidol (z.B. basenkatalysiert), Polymerisation von Epichlorhydrin (beispielsweise in Gegenwart äquimolarer Mengen einer Base wie NaOH) oder Polykondensation von Glycerin bereitgestellt werden.

Der erfindungsgemäße Polyglycerinpartialester ist erhältlich durch Veresterung eines Polyglycerins mit einer Carbonsäuremischung umfassend die Komponenten a) und b). Es ist erfindungsgemäß bevorzugt wenn die Komponenten a) und b) in Summe mindestens 80 Gew.-%, bevorzugt mindestens 90 Gew.-%, besonders bevorzugt mindestens 95 Gew.-%, bezogen auf die gesamte, eingesetzte Carbonsäuremischung ausmachen.

Es ist erfindungsgemäß bevorzugt, wenn die Dicarbonsäure im erfindungsgemäßen Polyglycerinpartialester ausgewählt ist aus aliphatischen, linearen Dicarbonsäuren, insbesondere Oxalsäure, Malonsäure, Tartronsäure, Bernsteinsäure, Maleinsäure, Weinsäure, Äpfelsäure, Fumarsäure, Sorbinsäure, $\alpha$-Ketoglutarsäure, Glutarsäure, Adipinsäure, Pimelinsäure, Korksäure, Azelainsäure, Sebacinsäure, Dodecandisäure, wobei Bernsteinsäure besonders bevorzugt ist.

Ein erfindungsgemäß bevorzugter Polyglycerinpartialester ist dadurch gekennzeichnet, dass die gesättigte Fettsäure ausgewählt ist aus unverzweigten, unsubstituierten Fettsäuren.

Ein erfindungsgemäß bevorzugter Polyglycerinpartialester ist dadurch gekennzeichnet, dass er eine Verseifungszahl von 70 bis 199 mg KOH / g, bevorzugt von 95 bis 190, besonders bevorzugt von 120 bis 180 mg KOH / g, aufweist. Die Bestimmung der Verseifungszahl erfolgt fachmännisch in Anlehnung an DGF C-V 3 oder DIN EN ISO 3681.

Ein erfindungsgemäß bevorzugter Polyglycerinpartialester ist dadurch gekennzeichnet, dass in der Veresterung das molare Verhältnis von a) zu b)

von 1,0 : 0,7 bis 1,0 : 3,0, bevorzugt

von 1,0 : 0,8 bis 1,0 : 2,0, besonders bevorzugt von

von 1,0 : 1,0 bis 1,0 : 1,6.

beträgt.

Ein erfindungsgemäß bevorzugter Polyglycerinpartialester ist dadurch gekennzeichnet, dass er bei 1 bar einen Trübungspunkt von 45 bis 75 °C, bevorzugt von 50 bis 65 °C, aufweist.

Die Bestimmung des Trübungspunkts erfolgt nach DIN EN 1890. Dazu werden 0,9 g des Polyglycerinpartialesters in einem 250 ml Becherglas eingewogen und mit 100 g 10%iger NaCl-Lösung versetzt. Diese Zusammensetzung wird erwärmt bis eine deutliche Trübung auftritt. Unter Umrühren mit dem Thermometer lässt man die Zusammensetzung abkühlen, bis diese wieder klar wird; diese Temperatur des Klarwerdens wird als "Trübungspunkt" definiert. Diese klare Zusammensetzung weist einen Trübungswert von NTU < 3 auf. Die Trübung wird mit einem HI88713 Trübungs-Labormessgerät (ISO 7027, Lichtdetektor: Silizium-Photozelle; Lichtquelle: Infrarot-LED) über die Durchlichtmethode im Normalmodus gegen Formazin gemessen und in NTU angegeben.

Ein erfindungsgemäß bevorzugter Polyglycerinpartialester ist dadurch gekennzeichnet, dass er eine Viskosität von 20 bis 200 Pa s, bevorzugt 50 bis 150 Pa s, bei 25 °C aufweist.

Die Viskosität wird mit einem Rheometer von Anton Paar, Modell MCR 301, Platte - Platte (40 mm) Geometrie bei einer Temperatur von 25 °C im Scherraten-Bereich von 0,1 s$^{-1}$ bis 1000 s$^{-1}$ bestimmt. Der hier angegebene Wert der Viskosität wird bei einer Scherrate von 10 s$^{-1}$ gemessen.

Es ist erfindungsgemäß bevorzugt, wenn der erfindungsgemäße Polyglycerinpartialester einen HLB-Wert gemäß Griffin, W. C.: Classification of surface active agents by HLB, J. Soc. Cosmet. Chem. 1, 1949, von 13 bis 17 aufweist.

Bevorzugt weist der erfindungsgemäße Polyglycerinpartialester eine Oberflächenspannung von weniger als 28 mN/m in 1,0 %iger wässriger Lösung bei 20 °C auf.

Die Oberflächenspannung wird dabei mit einem Pendant Drop Tensiometer OCA 35 von Dataphysics Instruments gemessen (www.dataphysics.de). Der angegebene Wert ist der Gleichgewichtswert.

Die Polyglycerinpartialester der vorliegenden Erfindung lassen sich durch klassische Veresterungsverfahren herstellen, anstelle der Carbonsäuren können natürlich auch die entsprechenden Carbonsäurederivate, beispielsweise deren Anhydride oder Carbonsäureester (wie Methyl- oder Ethylester) eingesetzt werden.

Ein weiterer Gegenstand der vorliegenden Erfindung ist eine Zusammensetzung enthaltend

den erfindungsgemäßen Polyglycerinpartialester und

10 bis 50 Gew.-%, bevorzugt 20 bis 40 Gew.-% 1,2- oder 1,3-Propandiol oder

5 bis 50 Gew.-%, %, bevorzugt 8 bis 30 % Gew.-%, Wasser,

wobei sich die Gewichtsprozente auf die Gesamtzusammensetzung beziehen und wobei die Zusammensetzungen eine Viskosität von 0,1 bis 10 Pa s, bevorzugt 0,5 bis 7,5 Pa s, bei 25 °C aufweisen.

Ein weiterer Gegenstand der vorliegenden Erfindung ist die Verwendung mindestens eines erfindungsgemäßen Polyglycerinpartialesters als Solubilisator, insbesondere von Ölen, insbesondere in kosmetischen oder pharmazeutischen Zubereitungen.

Insbesondere werden die Polyglycerinpartialester der vorliegenden Erfindung zur Solubilisierung von Ölen in Wasser, wässrigen und/oder tensidischen Formulierungen verwendet. Ebenso bevorzugt ist die Verwendung mindestens eines erfindungsgemäßen Polyglycerinpartialesters als Solubilisator von Ölen in aluminiumchlorohydrathaltigen kosmetischen Zubereitungen, insbesondere in Deo-Formulierungen.

Die erfindungsgemäße Verwendung findet insbesondere in polyetherfreien Zubereitungen statt. In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele:

*Beispiel 1: erfindungsgemäß*

Unter Stickstoffatmosphäre wurden 400 g Polyglycerin-3 (Hydroxylzahl = 1155 mg KOH / g) mit 80,0 g Capryl/Caprinsäure (0,30 Moläquiv.) und 55,0 g Bernsteinsäure (0,28 Moläquiv.) bei 240 °C gerührt bis eine Säurezahl von < 1 mg KOH / g erreicht war. Das im Verlauf der Reaktion gebildete Wasser wurde kontinuierlich abdestilliert. Nach Abkühlung auf Raumtemperatur lag das Reaktionsprodukt als hellgelbe, klare bis leicht trübe Flüssigkeit vor und wies die folgenden analytischen Kennzahlen auf: Viskosität: 120 Pa s; Viskosität, 75%ig in 1,2-Propandiol: 5,1 Pa s; Säurezahl: 0,2 mg KOH / g; Verseifungszahl: 157 mg KOH / g; Trübungspunkt: 56 °C.

*Beispiel 2: erfindungsgemäß*

Unter Stickstoffatmosphäre wurden 400 g Polyglycerin-3 (Hydroxylzahl = 1155 mg KOH / g) mit 85,0 g Capryl/Caprinsäure (0,32 Moläquiv.) und 55,0 g Bernsteinsäure (0,28 Moläquiv.) bei 240 °C gerührt bis eine Säurezahl von < 1 mg KOH / g erreicht war. Das im Verlauf der Reaktion gebildete Wasser wurde kontinuierlich abdestilliert. Nach Abkühlung auf Raumtemperatur lag das Reaktionsprodukt als hellgelbe, klare bis leicht trübe Flüssigkeit vor und wies die folgenden analytischen Kennzahlen auf: Viskosität: 96 Pa s; Säurezahl: 0,6 mg KOH / g; Verseifungszahl: 160 mg KOH / g; Trübungspunkt: 52 °C.

*Beispiel 3: erfindungsgemäß*

Unter Stickstoffatmosphäre wurden 400 g Polyglycerin-3 (Hydroxylzahl = 1155 mg KOH / g) mit 75,0 g Capryl/Caprinsäure (0,29 Moläquiv.) und 40,0 g Bernsteinsäure (0,20 Moläquiv.) bei 240 °C gerührt bis eine Säurezahl von < 1 mg KOH / g erreicht war. Das im Verlauf der Reaktion gebildete Wasser wurde kontinuierlich abdestilliert. Nach Abkühlung auf Raumtemperatur lag das Reaktionsprodukt als hellgelbe, klare bis leicht trübe Flüssigkeit vor und wies die folgenden analytischen Kennzahlen auf: Viskosität: 90 Pa s; Säurezahl: 0,3 mg KOH / g; Verseifungszahl: 131 mg KOH / g; Trübungspunkt: 50 °C.

*Beispiel 4: erfindungsgemäß*

Unter Stickstoffatmosphäre wurden 500 g Polyglycerin-3 (Hydroxylzahl = 1155 mg KOH / g) mit 93,8 g Capryl/Caprinsäure (0,29 Moläquiv.) und 25,0 g Bernsteinsäure (0,10 Moläquiv.) bei 240 °C gerührt bis eine Säurezahl von < 1 mg KOH / g erreicht war. Das im Verlauf der Reaktion gebildete Wasser wurde kontinuierlich abdestilliert. Nach Abkühlung auf Raumtemperatur lag das Reaktionsprodukt als hellgelbe, klare bis leicht trübe Flüssigkeit vor und wies die folgenden analytischen Kennzahlen auf: Viskosität: 123 Pa s; Säurezahl: 0,9 mg KOH / g; Verseifungszahl: 100 mg KOH / g; Trübungspunkt: 69 °C.

*Beispiel 5: erfindungsgemäß*

Unter Stickstoffatmosphäre wurden 300 g Polyglycerin-3 (Hydroxylzahl = 1155 mg KOH / g) mit 37,5 g Capryl/Caprinsäure (0,19 Moläquiv.) 11 h bei 180 °C gerührt. Nach Abkühlung auf 50 °C wurden 15,0 g Weinsäure (0,08 Moläquiv.) zugesetzt und weitere 14 h bei 180 °C gerührt. Das im Verlauf der Reaktion gebildete Wasser wurde kontinuierlich abdestilliert. Nach Abkühlung auf Raumtemperatur lag das Reaktionsprodukt als gelbe, klare bis leicht trübe Flüssigkeit vor und wies die folgenden analytischen Kennzahlen auf: Viskosität: 97 Pa s; Säurezahl: 2,0 mg KOH / g; Verseifungszahl: 73 mg KOH / g; Trübungspunkt: 51 °C.

*Beispiel 6: nicht erfindungsgemäß*

Unter Stickstoffatmosphäre wurden 400 g Polyglycerin-3 (Hydroxylzahl = 1155 mg KOH / g) mit 85,0 g Capryl/Caprinsäure (0,32 Moläquiv.) und 80,0 g Bernsteinsäure (0,40 Moläquiv.) bei 240 °C gerührt bis eine Säurezahl von < 1 mg KOH / g erreicht war. Das im Verlauf der Reaktion gebildete Wasser wurde kontinuierlich abdestilliert. Nach Abkühlung auf Raumtemperatur lag das Reaktionsprodukt als hellgelbe, klare bis leicht trübe Flüssigkeit vor und wies die folgenden analytischen Kennzahlen auf: Viskosität: 297 Pa s; Viskosität, 75%ig in 1,2-Propandiol: 7,8 Pa s; Säurezahl: 0,4 mg KOH / g; Verseifungszahl: 200 mg KOH / g; Trübungspunkt: 56 °C.

Beispiel 7:nicht erfindungsgemäß

Unter Stickstoffatmosphäre wurden 400 g Polyglycerin-3 (Hydroxylzahl = 1155 mg KOH / g) mit 85,0 g Capryl/Caprinsäure (0,32 Moläquiv.) und 150 g Bernsteinsäure (0,76 Moläquiv.) bei 240 °C gerührt bis eine Säurezahl von < 1 mg KOH / g erreicht war. Das im Verlauf der Reaktion gebildete Wasser wurde kontinuierlich abdestilliert. Nach Abkühlung auf Raumtemperatur lag das Reaktionsprodukt als hellgelbe, hochviskose, sehr klebrige Paste vor, so dass die meisten Kennzahlen aufgrund der schlechten Handhabbarkeit nicht bestimmt wurden: Säurezahl: 0,8 mg; Trübungspunkt: nicht bestimmbar, da Produkt nicht löslich.

*Beispiel 8: TEGOSOFT® PC 41, nicht erfindungsgemäß*

Standardsolubilisator, polyetherfrei. INCI: Polyglyceryl-4 Caprate. Verkaufsprodukt der Evonik Nutrition & Care GmbH.

*Beispiel 9: NATRAGEM™ S 140, nicht erfindungsgemäß*

Solubilisator für Öle, polyetherfrei. INCI: Polyglyceryl-4 Laurate/Sebacate (and) Polyglyceryl-6 Caprylate/Caprate (and) Aqua. Verkaufsprodukt von Croda.
Im Folgenden wurden die oben beschriebenen Produkte in kosmetischen Formulierungen ausgetestet. Die Formulierungsbestandteile sind in den Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben.

*Beispiel 10: Vergleich Beispiele 1 bis* 4 *gegen* 6, 8 *und 9 beim Lösungsvermögen von Ölen in Wasser*

Um das Lösungsvermögen der erfindungsgemäßen Polyglycerinpartialester zu untersuchen, wurden diese mit kosmetischen Ölen gemischt und mit Wasser versetzt. Als Öle wurden Rosmarinöl, Zitronenöl und das Parfumöl Spicy Herbs (alle vom Lieferanten Fragrance Resources) getestet. Es wurde untersucht, welcher Anteil an Solubilisator nötig ist, um 0,5% des jeweiligen Öls vollkommen klar in Wasser zu lösen. Mit vollkommen klar ist gemeint, dass die Lösung einen Trübungswert von NTU<3 aufweist. Für die Bestimmung des Lösungsvermögens wurde der Solubilisator (unterschiedliche Mengen) mit dem Öl (0,5 g) gut gemischt und dann unter Rühren langsam mit Wasser (auf 100 g aufgefüllt) versetzt. Es wurde eine halbe Stunde bei 25 °C gerührt. Ein "klares Gemisch" darf bei 25 °C innerhalb von 3 Tagen nicht wieder trüb werden.
In Tabelle 1 sind die erhaltenen Massenverhältnisse von Solubilisator zu Öl zusammengefasst, die nötig waren, klare Mischungen zu erhalten. In der letzten Spalte ist zudem notiert, wie leicht sich der Solubilisator formulieren ließ (+ = leichte Verarbeitbarkeit, - = schlechte Verarbeitbarkeit).

Tabelle 1: Solubilisator-zu-Öl Verhältnis, das für eine klare Lösung des Öls in Wasser nötig war

|  | Zitronenöl | Rosmarinöl | Spicy Herbs | Verarbeitung bei 20 °C |
|---|---|---|---|---|
| Polyglycerinpartialester Beispiel 1 | 4 : 1 | 3 : 1 | 6 : 1 | + |

(fortgesetzt)

| | Zitronenöl | Rosmarinöl | Spicy Herbs | Verarbeitung bei 20 °C |
|---|---|---|---|---|
| Polyglycerinpartialester Beispiel 2 | 4 : 1 | 3 : 1 | 6 : 1 | + |
| Polyglycerinpartialester Beispiel 3 | 6 : 1 | 5 : 1 | 4 : 1 | + |
| Polyglycerinpartialester Beispiel 4 | 8 : 1 | 4 : 1 | 7 : 1 | + |
| Polyglycerinpartialester Beispiel 6 (nicht erfindungsgemäß) | 9 : 1 | 4 : 1 | 7 : 1 | - |
| NATRAGEM™ S 140 (Beispiel 9, nicht erfindungsgemäß) | 14 : 1 (opak / nicht ganz klar!) | 7 : 1 (opak / nicht ganz klar!) | 18 : 1 (opak / nicht ganz klar!) | + |
| TEGOSOFT® PC 41 (Beispiel 8, nicht erfindungsgemäß) | 16 : 1 | 14 : 1 | 15 : 1 | ++ |

Anhand der Ergebnisse in Tabelle 1 ist ersichtlich, dass die erfindungsgemäßen Polyglycerinpartialester 1, 2, 3 und 4 fast immer bessere Solubilisierungseigenschaften aufweisen als die Vergleichsbeispiele 6 und v.a. als 8 und 9. Das den Produkten 1-4 sehr ähnliche Produkt 6 (mit dem hohen Dicarbonsäureanteil im Polyglycerinester) hat eine deutlich höhere Viskosität und ist neben den etwas schlechteren Solublisierungseigenschaften v.a. auch deutlich schlechter verarbeitbar als die Produkte 1-4. Für die Verarbeitung von Produkt 6 ist ein Erwärmen des Ansatzes vorzuziehen, da sonst die Mischung mit dem Öl nicht gut einheitlich herzustellen ist. Ein Erwärmen ist bei den anderen Produkten nicht nötig. Bei dem ebenfalls sehr ähnlichen Produkt 8 des Stands der Technik fällt darüber hinaus auf, dass dieses die Öle nicht vollkommen klar in Wasser zu lösen vermag. Die Ansätze mit Produkt 8 sind immer etwas opak (auch mit wesentlich höheren Solubilisator-Überschüssen als oben in der Tabelle angegeben).

*Beispiel 11: Vergleich Beispiel 2 gegen 8 und 9 beim Lösungsvermögen von Ölen in Aluminiumchlorohydrat-haltiger Deo-Roll-on-Formulierung*

Neben den in Beispiel 10 gezeigten Lösungseigenschaften der erfindungsgemäßen Polyglycerinpartialester für Öle in Wasser wurde das Lösungsvermögen für Öle auch in einer besonders anspruchsvollen Deo-Formulierung (siehe Tabelle 2) mit einem hohen Gehalt an Aluminiumchlorohydrat (ACH) untersucht.

Tabelle 2: Formulierung Y zur Beurteilung der Solubilisierungseigenschaften in einer hoch Aluminiumchlorohydrat-haltigen Deo-Formulierung

| Phase | Inhaltsstoff | Anteil in % |
|---|---|---|
| A | Hydroxyethylcellulose, (Natrosol 250 HR, Ashland) | 1,00% |
| | Water | ad 100,00% |
| B | Aluminum Chlorohydrate, 50%ig in Wasser, (Locron L, Clariant) | 40,00% |
| | Phenoxyethanol; Methylisothiazolinone, (Neolone PE, Konservierungsmittel, Dow) | 0,45% |
| C | Fragrance oil (Spicy Herb bzw. Pink Grapefruit, beide von Fragrance Resources) | 1,00% |
| | Solubilizer | q.s. |

Dazu wurde der jeweilige Solubilisator mit 1,0 g der Parfumöle Spicy Herb oder Pink Grapefruit (beide von Fragrance Resources) bei Raumtemperatur für 5 min gemischt. Parallel dazu wurde 1,0 g Hydroxyethylcellulose unter Rühren langsam zu Wasser bei 45 °C gegeben und unter Rühren innerhalb ca. 90 - 120 min klar gelöst (Phase A). Phase B mit dem Aluminiumchlorohydrat wurde dann unter Rühren zu Phase A gegeben. Unter Rühren wurde zuletzt die Mischung aus Solubilisator und Parfumöl mit dem Gemisch aus Phasen A + B langsam versetzt.
Es wurde untersucht, welcher Anteil an Solubilisator nötig ist, um 1,0% des Parfumöls vollkommen klar in dem Deo-System zu lösen.

In Tabelle 3 sind die erhaltenen Massenverhältnisse von Solubilisator-zu-Öl zusammengefasst, die nötig waren, um klare Mischungen zu erhalten.

Tabelle 3: Solubilisator-zu-Öl Verhältnis, das für eine klare Lösung des jeweiligen Parfumöls in der Deo-Formulierung Y nötig war

|  | Spicy Herbs | Pink Grapefruit |
|---|---|---|
| Polyglycerinpartialester Beispiel 2 | 6 : 1 | 8 : 1 |
| NATRAGEM™ S 140 (Beispiel 9, nicht erfindungsgemäß) | >20 : 1 (Phasenseparation über Nacht) | >20 : 1 (Phasenseparation über Nacht) |
| TEGOSOFT® PC 41 (Beispiel 8, nicht erfindungsgemäß) | > 20 : 1 | > 20 : 1 |

Die Ergebnisse in Tabelle 3 zeigen, dass der erfindungsgemäße Polyglycerinpartialester Beispiel 2 überraschenderweise sehr deutlich verbesserte Lösungsvermittlereigenschaften für die Öle in dieser ACH-Formulierung aufweist im Vergleich zu den Vergleichsprodukten NATRAGEM™ S 140 und TEGOSOFT® PC 41. Selbst bei einem Verhältnis von 20:1 schaffen die Vergleichsbeispiele es nicht, die Öle klar in der Formulierung zu lösen.

*Beispiel 12: Vergleich Beispiel 1 gegen 6 und 9 bei Hautpflegevermögen und Schaumeigenschaften*

Zur Bewertung der Hautpflegeleistung und der Schaumeigenschaften des erfindungsgemäßen Polyglycerinpartialesters Beispiel 1 in wässrigen, tensidischen Formulierungen wurde ein sensorischer Handwaschtest im Vergleich zu den Vergleichsbeispielen 6 und 9 nach dem Stand der Technik durchgeführt.

Eine Gruppe bestehend aus 10 trainierten Prüfpersonen wusch sich dabei definiert die Hände und bewertete Schaumeigenschaften und Hautgefühl anhand einer Notenskala von 1 (schlecht) bis 5 (sehr gut).

Die Produkte wurden jeweils in einer standardisierten Tensidformulierung formuliert, mit dem Standard-Tensidsystem 9% aktiv Sodium Laureth Sulfate und 3% aktiv Cocamidopropyl Betaine (Tabelle 4).

Tabelle 4: Testformulierung für den sensorischen Handwaschtest:

| Formulierungsbeispiel | U | V | W | X |
|---|---|---|---|---|
| Texapon® NSO-IS, 28%-ig, (INCI: Sodium Laureth Sulfate, BASF) | 32,0% | 32,0% | 32,0% | 32,0% |
| TEGO® Betain F 50, 38%-ig, (INCI: Cocamidopropyl Betaine, Evonik) | 8,0% | 8,0% | 8,0% | 8,0% |
| Sodium Chloride | 1,5% | 1,5% | 1,5% | 1,5% |
| Citric Acid | 0,2% | 0,2% | 0,2% | 0,2% |
| Water, demineralized | 58,3% | 55,3% | 55,3% | 54,55% |
| Polyglycerinpartialester Beispiel 1 | - | 3,0% | - | - |
| Polyglycerinpartialester Beispiel 6 (nicht erfindungsgemäß) | - | - | 3,0% | - |
| NATRAGEM® S 140, 80%-ig mit 20% Wasser, (Beispiel 9, nicht erfindungsgemäß) |  |  |  | 3,75% |

Die sensorischen Testergebnisse sind in Tabelle 5 zusammengefasst.

Tab. 5: Ergebnisse des Handwaschtests:

| Testformulierung | U | V | W | X |
|---|---|---|---|---|
| Anschäumverhalten | 3,3 | 3,6 | 3,6 | 3,5 |
| Schaumvolumen | 3,0 | 3,1 | 3,1 | 3,1 |
| Schaumcremigkeit | 2,7 | 3,9 | 3,9 | 4,0 |
| Hautgefühl während des Waschens | 2,9 | **3,2** | 3,0 | 3,0 |

(fortgesetzt)

| Testformulierung | U | V | W | X |
|---|---|---|---|---|
| Abwaschbarkeit | 3,5 | 3,6 | 3,6 | 3,5 |
| Hautglätte | 2,0 | **2,8** | 2,6 | 2,5 |
| Hautweichheit | 2,3 | **3,0** | 2,7 | 2,7 |
| Hautglätte nach 3 min. | 3,0 | **3,6** | 3,3 | 3,2 |
| Hautweichheit nach 3 min. | 2,9 | 3,6 | 3,4 | 3,5 |

Anhand der Testergebnisse in Tabelle 5 wird ersichtlich, dass die erfindungsgemäße Formulierung V unter Verwendung des erfindungsgemäßen Polyglycerinpartialesters Beispiel 1 überraschenderweise in den Applikationseigenschaften Hautglätte und Weichheit im Vergleich zu den Vergleichsformulierungen W und X nach dem Stand der Technik überlegen ist. Auch das Hautgefühl während des Waschens ist mit Formulierung V am besten. Vor diesem Hintergrund sind die Ergebnisse der erfindungsgemäßen Formulierung V als sehr gut zu bezeichnen und zeigen eine deutliche Verbesserung gegenüber dem Stand der Technik.

*Beispiel-Formulierungen:*

Die in den nachfolgenden Tabellen angegebenen Formulierungsbeispiele zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet. Falls eine zweiphasige Herstellung erforderlich ist, werden die beiden Phasen mit A und B in den angegebenen Tabellen gekennzeichnet. Bei drei- bzw. mehrphasigen Prozessen werden die Phasen mit A, B und C etc. benannt. Wenn nicht anders angegeben handelt es sich bei den Angaben in den Tabellen um Angaben in Gew.-%. In den folgenden Formulierungsbeispielen sind die Angaben bzw. Gew.-% auf den jeweiligen Aktivstoff bezogen. Einige Produkte sind jedoch kommerziell als Lösungen in v.a. Wasser erhältlich, so dass in diesen Fällen dementsprechend je nach Aktivgehalt mehr von den kommerziellen Produkten eingesetzt wurde. "Produkt Beispiel 1 bis 9" entsprechen den "Polyglycerinpartialestern Beispiel 1 - 9".

Tab. 6: Creme-Bad

| | |
|---|---|
| Water | ad 100,0% |
| Sodium Laureth Sulfate | 8,0% |
| Coco-Glucoside | 4,0% |
| Cocamidopropyl Betaine | 4,0% |
| Produkt Beispiel 2 | 1,5% |
| PEG-18 Glyceryl Oleate/Cocoate | 2,0% |
| Perfume Spicy Herbs | 0,2% |
| Polyglyceryl-4 Caprate | 0,5% |
| Citrus Aurantifolia (Lime) Oil | 0,2% |
| Linalool | 0,1% |
| Coumarin | 0,1% |
| Glycerin | 0,5% |
| Glycol Distearate | 0,5% |
| Styrene/Acrylates Copolymer | 0,2% |
| Tocopherol | 0,1% |
| Preservative | q.s. |
| Citric Acid | ad pH 5,2 |

Tab. 7: Creme-Dusche

| | |
|---|---|
| Water | ad 100,0% |
| Glycerin | 4,0% |
| Sodium Laureth Sulfate | 4,0% |
| Cocamidopropyl Betaine | 3,5% |
| Produkt Beispiel 3 | 1,8% |
| Coco-Glucoside | 1,8% |
| Ricinus Communis Seed Oil (Seed) | 0,2% |
| Glyceryl Oleate | 0,5% |
| Polyglyceryl-6 Caprylate; Polyglyceryl-3 Cocoate; Polyglyceryl-4 Caprate; Polyglyceryl-6 Ricinoleate | 1,0% |
| Argania Spinosa Kernel Oil | 0,1% |
| Butyrospermum Parkii Butter Extract | 0,1% |
| Limonene | 0,1% |
| Perfume | 0,2% |
| Acrylates/C10-30 Alkyl Acrylate Crosspolymer | 0,2% |
| Hydroxypropyl Methylcellulose | 0,2% |
| Styrene/Acrylates Copolymer | 0,2% |
| Sodium Hydroxide | 0,2% |
| Glycol Distearate | 0,4% |
| Silica | 0,2% |
| Tocopherol | 0,1% |
| Preservative | q.s. |
| Citric Acid | ad pH 5,2 |

Tab. 8: Badeöl

| | |
|---|---|
| Water | ad 100,0% |
| Sodium Laureth Sulfate | 6,0% |
| Cocamidopropyl Betaine | 6,0% |
| Cocamide DEA | 2,5% |
| Sodium Trideceth Sulfate | 2,2% |
| Produkt Beispiel 2 | 1,0% |
| Perfume | 0,5% |
| PEG-40 Hydrogenated Castor Oil | 0,2% |
| Trideceth-9 | 0,2% |
| Sodium Lauroamphoacetate | 0,5% |
| Benzophenone-4 | 0,2% |
| Cocamide MEA | 0,4% |
| Propylene Glycol | 0,5% |
| Disodium EDTA | 0,1% |

(fortgesetzt)

| | |
|---|---|
| Sodium Chloride | 0,6% |
| Glycerin | 0,5% |
| Benzyl Alcohol | 0,4% |
| Sodium Cocoyl Glutamate | 0,4% |
| Phenoxyethanol | 0,2% |
| Xanthan Gum | 0,2% |
| Carbomer | 0,2% |
| Lactic Acid | 0,3% |
| Magnesium Chloride | 0,1% |
| Coumarin | 0,1% |
| Citric Acid | ad pH 5,2 |
| Preservative | q.s. |

Tab. 9: Creme-Dusche

| | |
|---|---|
| Water | ad 100,0% |
| Sodium Laureth Sulfate | 8,0% |
| Glycerin | 2,5% |
| Cocamidopropyl Betaine | 2,5% |
| Produkt Beispiel 3 | 2,0% |
| Decyl Glucoside | 1,5% |
| Perfume | q.s. |
| Sodium Chloride | 1,5% |
| PEG-40 Hydrogenated Castor Oil | 0,5% |
| Glycine Soja Oil | 0,1% |
| Helianthus Annuus Seed Oil | 0,1% |
| Lecithin | 0,2% |
| Coco-Glucoside | 0,5% |
| Glyceryl Oleate | 0,2% |
| Coumarin | 0,1% |
| Preservative | q.s. |

Tab. 10: Körpershampoo

| | | |
|---|---|---|
| Phase A | Produkt Beispiel 2 | 3,0% |
| | Salvia Officinalis (Sage) Oil | 0,3% |
| | Perfume | 0,1% |
| Phase B | Sodium Cocoamphoacetate | 3,5% |
| Phase C | Water | ad 100,0% |
| | Acrylates / C10-30 Alkyl Acrylate Crosspolymer | 0,9% |

(fortgesetzt)

| Phase D | Sodium Lauroyl Methyl Isethionate | 5,0% |
|---|---|---|
| | Capryl/Capramidopropyl Betaine | 1,5% |
| | Citric Acid | 1,2% |
| Phase E | Water | 10,0% |
| | Polyquaternium-7 | 0,3% |
| | Preservative | q.s. |

Tab. 11: Körpershampoo

| Phase A | Produkt Beispiel 1 | 4,0% |
|---|---|---|
| | Lavandula Angustifolia (Lavender) Oil | 0,2% |
| | Perfume | 0,2% |
| Phase B | Water | ad 100,0% |
| Phase C | Sodium Cocoamphoacetate | 4,0% |
| Phase D | Water | 30,0% |
| | Acrylates/Beheneth-25 Methacrylate Copolymer | 1,7% |
| | Sodium Lauroyl Methyl Isethionate | 4,0% |
| | Disodium Lauryl Sulfosuccinate | 1,7% |
| Phase E | Preservative | q.s. |

Tab. 12: Shampoo

| Phase A | Produkt Beispiel 2 | 3,0% |
|---|---|---|
| | Orange Oil | 0,3% |
| | Perfume | 0,1% |
| Phase B | Water | ad 100,0% |
| Phase C | Disodium Cocoamphodiacetate | 5,0% |
| Phase D | Glycerin | 1,0% |
| | Xanthan Gum | 0,7% |
| | Water | 25,0% |
| Phase E | Water | 10,0% |
| | Acrylates/Beheneth-25 Methacrylate Copolymer | 2,0% |
| Phase F | Water | 10,0% |
| | Polyquaternium-10 | 0,3% |
| Phase G | Cocamidopropyl Betaine | 5,0% |
| | Preservative | q.s. |

Tab. 13: Shampoo

| Phase A | Produkt Beispiel 2 | 5,0% |
|---|---|---|
| | Lemongrass Oil | 0,5% |

(fortgesetzt)

| Phase B | Water | ad 100,0% |
|---|---|---|
| Phase C | Perfume | 0,2% |
| | Polyglyceryl-4 Caprate | 2,0% |
| Phase D | Water | 20,0% |
| Phase E | Sodium Laureth Sulfate | 8,0% |
| Phase F | Water | 10,0% |
| | Cocamidopropyl Betaine | 4,0% |
| | PEG-120 Methyl Glucose Dioleate | 1,2% |
| Phase G | Water | 10,0% |
| | Sodium Chloride | 0,9% |
| | Polyquaternium-10 | 0,2% |
| Phase H | Citric Acid | ad pH 5,5 |
| Phase I | Preservative | q.s. |

Tab. 14: Duschgel

| Phase A | Produkt Beispiel 2 | 3,0% |
|---|---|---|
| | Mentha Piperita (Peppermint) Oil | 0,2% |
| | Rosemary Oil | 0,2% |
| | Perfume | 0,1% |
| Phase B | Water | ad 100,0% |
| Phase C | Sodium Cocoamphoacetate | 5,5% |
| Phase D | Lauryl Glucoside | 4,5% |
| Phase E | Coco-Glucoside | 1,3% |
| Phase F | Sodium/Disodium Cocoyl Glutamate | 3,5% |
| | Water | 10,0% |
| | Glycerin | 0,8% |
| Phase G | Water | 10,0% |
| | Xanthan Gum | 2,2% |
| Phase H | Citric Acid | ad pH 6,0 |
| Phase I | Preservative | q.s. |

Tab. 15: Shampoo

| Phase A | Produkt Beispiel 1 | 4,0% |
|---|---|---|
| | Isopropyl Myristate | 0,2% |
| | Perfume | 0,1% |
| Phase B | Water | ad 100,0% |
| Phase C | Sodium Lauryl Sulfate | 9,0% |
| Phase D | Cocamidopropyl Betaine | 3,0% |

**EP 3 168 251 B1**

(fortgesetzt)

| Phase E | Cocamide MEA | 2,0% |
|---|---|---|
| | Xanthan Gum | 0,2% |
| | Water | 10,0% |
| Phase F | Water | 10,0% |
| | Polyquaternium-10 | 0,2% |
| Phase G | Citric Acid | ad pH 5,5 |
| Phase H | Preservative | q.s. |

Tab. 16: Cleansing Oil Shampoo

| | |
|---|---|
| Water | ad 100,0% |
| Sodium Laureth Sulfate | 6,0% |
| MIPA-Laureth Sulfate | 3,0% |
| Sodium Chloride | 2,5% |
| Cocamidopropyl Betaine | 2,5% |
| Polyglyceryl-6 Caprylate; Polyglyceryl-3 Cocoate; Polyglyceryl-4 Caprate; Polyglyceryl-6 Ricinoleate | 2,5% |
| Glycerin | 2,5% |
| PEG-18 Castor Oil Dioleate | 2,0% |
| Propylene Glycol; PEG-55 Propylene Glycol Oleate | 2,0% |
| Produkt Beispiel 2 | 2,0% |
| Laureth-5 Carboxylic Acid | 1,0% |
| Persea Gratissima (Avocado) Oil | 0,5% |
| Sodium Benzoate | 0,7% |
| Salicylic Acid | 0,3% |
| Linalool | 0,2% |
| alpha-Isomethyl Ionone | 0,1% |
| Limonene | 0,1% |
| Camellia Oleifera Seed Oil | 0,1% |
| Citric Acid | ad pH 5,0 |
| Perfume, Dyes | q.s. |

Tab. 17: Shower Cream

| | |
|---|---|
| Water | ad 100% |
| Glycerin | 7,0% |
| Glycine Soja Oil | 3,0% |
| Lauryl Glucoside | 3,0% |
| Sodium Coco-Sulfate | 3,0% |
| Produkt Beispiel 2 | 2,5% |
| Alcohol | 1,5% |

**14**

(fortgesetzt)

| Xanthan Gum | 1,5% |
|---|---|
| Butyrospermum Parkii Butter Extract | 1,0% |
| Sodium Cetearyl Sulfate | 1,0% |
| Sodium Cocoyl Glutamate | 1,0% |
| Disodium Cocoyl Glutamate | 1,0% |
| Tocopherol | 0,1% |
| Helianthus Annuus Seed Oil | 0,3% |
| Limonene | 0,1% |
| Benzyl Salicylate | 0,1% |
| Linalool | 0,1% |
| Dyes | q.s. |

Tab. 18: Shower Gel

| Water | ad 100% |
|---|---|
| Sodium Coco-Sulfate | 5,0% |
| Glycerin | 4,0% |
| Lauryl Glucoside | 4,0% |
| Sodium Lactate | 2,5% |
| Produkt Beispiel 3 | 2,0% |
| Polyglyceryl-4 Caprate | 2,0% |
| Sodium Cocoyl Glutamate | 2,0% |
| Disodium Cocoyl Glutamate | 1,0% |
| Alcohol | 1,0% |
| Prunus Cerasus Fruit Extract | 1,0% |
| Limonene | 0,1% |
| Coumarin | 0,2% |
| Linalool | 0,1% |
| Citral | 0,1% |
| Dyes | q.s. |

Tab. 19: Liquid Soap

| Water | ad 100% |
|---|---|
| Glycerin | 4,0% |
| Alcohol | 4,0% |
| Sodium Coco-Sulfate | 3,0% |
| Lauryl Glucoside | 3,0% |
| Produkt Beispiel 1 | 2,0% |
| Xanthan Gum | 1,5% |

(fortgesetzt)

| | |
|---|---|
| Mangifera Indica (Mango) Fruit Extract | 0,5% |
| Limonene | 0,1% |
| Linalool | 0,1% |
| Dyes | q.s. |

Tab. 20: Shampoo for Children

| | |
|---|---|
| Water | ad 100% |
| Sodium Coco Sulfate | 7,0% |
| Decyl Glucoside | 4,0% |
| Lactis Proteinum | 2,5% |
| Sorbitan Caprylate | 2,0% |
| Produkt Beispiel 2 | 2,0% |
| Glycerin | 2,0% |
| Sodium Lactate | 2,0% |
| Alcohol | 2,0% |
| Hydrolyzed Wheat Protein | 0,3% |
| Hydrolyzed Wheat Starch | 0,3% |
| Sodium Chloride | 0,9% |
| Limonene | 0,1% |
| Citral | 0,1% |
| Phenethyl Alcohol | 0,1% |
| Dyes | q.s. |

Tab. 21: Cream Soap

| | |
|---|---|
| Water | ad 100% |
| Alcohol | 7,0% |
| Coco-Glucoside | 5,0% |
| Glycerin | 5,0% |
| Produkt Beispiel 1 | 2,5% |
| Disodium Cocoyl Glutamate | 2,5% |
| Xanthan Gum | 1,5% |
| Citric Acid | ad pH 5,5 |
| Malva Sylvestris Leaf Extract | 1,0% |
| Glyceryl Oleate | 1,0% |
| Sodium Cocoyl Glutamate | 0,8% |
| Linalool | 0,1% |
| Limonene | 0,1% |
| Dyes | q.s. |

EP 3 168 251 B1

Tab. 22: Crème Dusche

| Water | ad 100% |
|---|---|
| Ammonium Lauryl Sulfate | 11,0% |
| Produkt Beispiel 2 | 3,5% |
| Aloe Barbadensis Leaf Juice | 1,5% |
| Cocamidopropyl Betaine | 1,5% |
| Decyl Glucoside | 1,5% |
| Glycerin | 1,0% |
| Prunus Amygdalus Dulcis Oil | 0,4% |
| Glyceryl Oleate | 0,2% |
| Lauryl Glucoside | 0,3% |
| Coco-Glucoside | 0,4% |
| Benzyl Alcohol | 0,2% |
| Benzoic Acid | 0,2% |
| Dehydroacetic Acid | 0,1% |
| Sodium Benzoate | 0,3% |
| Potassium Sorbate | 0,2% |
| Tocopherol | 0,1% |
| Citric Acid | ad pH 4,9 |
| Perfume, Dyes | q.s. |

Tab. 23: Creme Dusche

| Water | ad 100% |
|---|---|
| Sodium Laureth Sulfate | 9,0% |
| Produkt Beispiel 2 | 3,0% |
| Cocamidopropyl Betaine | 3,0% |
| Glycerin | 1,0% |
| Glucose | 0,5% |
| Prunus Amygdalus Dulcis Oil | 0,5% |
| Sodium Chloride | 0,5% |
| Polyquaternium-7 | 0,3% |
| Styrene/Acrylates Copolymer | 0,3% |
| PEG-200 Hydrogenated Glyceryl Palmate; PEG-7 Glyceryl Cocoate | 0,6% |
| Citric Acid | ad pH 5,5 |
| Perfume, Dyes | q.s. |

Tab. 24: Pflegedusche

| Water | ad 100% |
|---|---|
| Sodium Laureth Sulfate | 9,5% |

17

(fortgesetzt)

| | |
|---|---|
| Sodium Hydroxypropyl Starch Phosphate | 2,5% |
| Produkt Beispiel 2 | 2,0% |
| Cocamidopropyl Betaine | 2,5% |
| Petrolatum | 0,4% |
| Sodium Cocoyl Glycinate | 0,9% |
| Lauric Acid | 0,5% |
| Sodium Lauroyl Isethionate | 0,7% |
| Glycerin | 0,5% |
| Helianthus Annuus Seed Oil | 0,2% |
| Olea Europaea Fruit Oil | 0,1% |
| Sodium Chloride | 0,5% |
| Stearic Acid | 0,3% |
| Guar Hydroxypropyltrimonium Chloride | 0,3% |
| Sodium Isethionate | 0,1% |
| Tetrasodium EDTA | 0,1% |
| Alumina | 0,1% |
| Citric Acid | ad pH 5,5 |
| Perfumes, Dyes, Preservatives | q.s. |

Tab. 25: Creme Dusche

| | |
|---|---|
| Water | ad 100% |
| Sodium Coco-Sulfate | 15,0% |
| Glycerin | 3,5% |
| Produkt Beispiel 3 | 3,5% |
| Glycine Soja Oil | 0,3% |
| Coco-Glucoside | 0,9% |
| Caprylic/Capric Triglyceride | 0,1% |
| Xanthan Gum | 0,8% |
| Prunus Amygdalus Dulcis Oil | 0,1% |
| Sodium Cocoyl Glutamate | 0,3% |
| Disodium Cocoyl Glutamate | 0,5% |
| Sodium Cetearyl Sulfate | 0,2% |
| Tocopherol | 0,1% |
| Helianthus Annuus Seed Oil | 0,1% |
| Alcohol | 0,8% |
| Citral | 0,1% |
| Geraniol | 0,1% |
| Limonene | 0,1% |

(fortgesetzt)

| | |
|---|---|
| Linalool | 0,1% |
| Citric Acid | ad pH 5,3 |
| Perfume, Dyes | q.s. |

Tab. 26: Pampering Oil Bath

| | |
|---|---|
| Water | ad 100,0% |
| Glycine Soja Oil | 20,0% |
| Produkt Beispiel 1 | 10,0% |
| Polyglyceryl-3 Palmitate | 4,5% |
| Glyceryl Caprylate | 4,5% |
| Simmondsia Chinensis Seed Oil | 1,5% |
| Prunus Amygdalus Dulcis (Sweet Almond) Oil | 1,0% |
| Triticum Vulgare Germ Oil | 1,0% |
| Tocopherol | 0,2% |
| Limonene | 0,1% |
| Citral | 0,1% |
| Dyes | q.s. |

Tab. 27: Deo

| | | |
|---|---|---|
| Phase A | Produkt Beispiel 2 | 3,0% |
| | Isopropyl Palmitate | 0,1% |
| | Rosemary Oil | 0,1% |
| | Perfume | 0,1% |
| Phase B | Phenoxyethanol | 0,5% |
| | Caprylyl Glycol | 0,2% |
| Phase C | Water | 50,0% |
| | Hydroxethyl Cellulose | 0,7% |
| | Sodium Hydroxide (10% in water) | 0,2% |
| Phase D | Aluminium Chlorohydrate | 19,0% |
| Phase E | Water | ad 100,0% |

Tab. 28: Deo

| | | |
|---|---|---|
| Phase A | Phenoxyethanol | 0,5% |
| | Methylisothiazolinone | 0,001% |
| | Aluminium Chlorohydrate | 20,0% |
| Phase B | Hydroxethyl Cellulose | 1,0% |
| | Water | ad 100,0% |

(fortgesetzt)

| Phase C | Produkt Beispiel 3 | 1,5% |
|---|---|---|
| | Perfume Spicy Herbs | 0,3% |

Tab. 29: Deo

| Phase A | Produkt Beispiel 1 | 2,0% |
|---|---|---|
| | Perfume Pink Grapefruit | 0,2% |
| Phase B | Phenoxyethanol | 0,5% |
| | Caprylyl Glycol | 0,2% |
| Phase C | Water | 50,0% |
| | Hydroxethyl Cellulose | 0,75% |
| | Sodium Hydroxide (10% in water) | 0,25% |
| Phase D | Aluminium Chlorohydrate | 10,0% |
| Phase E | Water | ad 100,0% |

Tab. 30: Antitranspirant Deo Roll-On

| Phase A | Polyglyceryl-6 Caprylate; Polyglyceryl-3 Cocoate; Polyglyceryl-4 Caprate; Polyglyceryl-6 Ricinoleate | 4,0% |
|---|---|---|
| | Decyl Oleate | 0,1% |
| | Aloe Barbadensis Leaf Extract | 0,1% |
| | Glycine Soja Oil | 0,1% |
| Phase B | Produkt Beispiel 1 | 2,8% |
| | Perfume | 0,1% |
| | Distearyl Ether | 0,3% |
| | Stearyl Alcohol | 0,1% |
| Phase C | Aluminium Chlorohydrate | 10,0% |
| | Water | ad 100,0% |
| Phase D | Phenoxyethanol | 0,6% |
| | Ethylhexylglycerin | 0,2% |

Tab. 31: Anti-Transpirant Deo

| Phase A | Produkt Beispiel 2 | 3,5% |
|---|---|---|
| | Dicaprylyl Ether | 0,2% |
| | Geraniol | 0,1% |
| | Linalool | 0,1% |
| | Perfume Spicy Herbs | 0,1% |

(fortgesetzt)

| Phase B | Propylene Glycol | 1,0% |
|---|---|---|
| | Butylene Glycol | 0,5% |
| | Water | 5,0% |
| | Palmitamidopropyltrimonium Chloride | 1,5% |
| Phase C | Water | 50,0% |
| | Hydroxethyl Cellulose | 0,8% |
| | Sodium Hydroxide (10% in water) | 0,3% |
| Phase D | Aluminium Chlorohydrate | 15,0% |
| Phase E | Water | ad 100,0% |

Tab. 32: Deo

| Phase A | Produkt Beispiel 1 | 2,8% |
|---|---|---|
| | Dicaprylyl Ether | 0,2% |
| | Isoceteth-20 | 0,5% |
| | Geraniol | 0,1% |
| | Allantoin | 0,1% |
| | Linalool | 0,1% |
| | Limonene | 0,1% |
| | Butylene Glycol | 0,8% |
| | Perfume Pink Grapefruit | 0,1% |
| Phase B | Propylene Glycol | 1,0% |
| | Water | 5,0% |
| | Palmitamidopropyltrimonium Chloride | 1,5% |
| Phase C | Water | 50,0% |
| | Glyceryl Isostearate | 0,5% |
| | PEG-150 Distearate | 0,3% |
| Phase D | Aluminium Chlorohydrate | 10,0% |
| Phase E | Water | ad 100,0% |

Tab. 33: Formulierung für Wet Wipes

| | |
|---|---|
| Butylene Glycol | 1,0% |
| Glycerin | 1,0% |
| Produkt Beispiel 1 | 1,5% |
| Silicone Quaternium-22; Polyglycerin-3 Caprate; Dipropylene Glycol; Cocamidopropyl Betaine | 0,5% |
| Allantoin | 0,2% |
| Maltodextrin | 0,5% |
| Chamomilla Recutita Extract | 0,1% |
| Phenoxyethanol; Ethylhexylglycerin | 0,7% |

(fortgesetzt)

| | |
|---|---|
| Perfume | q.s. |
| Water | ad 100,0% |
| Citric Acid, 30% | ad pH 5,5 |

Tab. 34: Lösung für Wet Wipes

| | |
|---|---|
| Produkt Beispiel 2 | 1,5% |
| Perfume | 0,2% |
| Glycerin | 2,0% |
| Sodium Lactate; Sodium PCA; Glycine; Fructose; Urea; Niacinamide; Inositol; Sodium Benzoate; Lactic Acid | 0,1% |
| Water | ad 100,0% |
| Preservative | q.s. |

Tab. 35: Lösung für Wet Wipes

| | |
|---|---|
| Produkt Beispiel 3 | 3,0% |
| Polyglyceryl-4 Caprate | 2,0% |
| Isopropyl Myristate | 0,3% |
| Phenoxyethanol; Methylparaben; Ethylparaben; Butylparaben; Propylparaben; Isobutylparaben | 0,2% |
| Perfume | 0,1% |
| Propylene Glycol | 2,0% |
| Water | ad 100,0% |
| Cetrimonium Bromide | 0,1% |

Tab. 36: Lösung für Wet Wipes

| | |
|---|---|
| Produkt Beispiel 2 | 3,0% |
| Aloe Barbadensis Leaf Extract | 0,3% |
| Disodium Cocoamphodiacetate | 0,5% |
| Perfume | 0,2% |
| Propylene Glycol | 2,5% |
| Hydrolyzed Silk | 0,1% |
| Caprylyl/Capryl Glucoside | 1,0% |
| Water | ad 100,0% |
| Phenoxyethanol | 0,5% |
| Dehydroacetic Acid | 0,1% |
| Benzoic Acid | 0,1% |
| Sodium Benzoate | 0,4% |

Tab. 37: Make-up Remover

| | |
|---|---|
| Sodium Cocoamphopropionate | 5,0% |
| Propylene Glycol | 35,0% |
| Produkt Beispiel 1 | 30,0% |
| Glycerin | 30,0% |
| Preservative | q.s. |

Tab. 38: Make-up Remover

| | |
|---|---|
| Cocamidopropyl Betaine | 7,0% |
| Water | ad 100,0% |
| Produkt Beispiel 2 | 4,0% |
| Glycerin | 8,0% |
| Citric Acid | ad pH 5,5 |
| Preservative | q.s. |

Tab. 39: Make-up Remover

| | |
|---|---|
| Capryl/Capramidopropyl Betaine | 2,0% |
| Water | ad 100,0% |
| Produkt Beispiel 2 | 3,0% |
| Glycerin | 3,0% |
| Citric Acid | ad pH 5,5 |
| Preservative | q.s. |

Tab. 40: Make-up Remover

| | |
|---|---|
| Polyglyceryl-4 Caprate | 1,0% |
| Water | ad 100,0% |
| Produkt Beispiel 2 | 1,5% |
| Polyglyceryl-6 Caprylate; Polyglyceryl-3 Cocoate; Polyglyceryl-4 Caprate; Polyglyceryl-6 Ricinoleate | 1,2% |
| Propylene Glycol | 1,0% |
| Glycerin | 2,5% |
| Citric Acid | ad pH 5,5 |
| Preservative | q.s. |

Tab. 41: O/W Make-up remover wipe

| | | |
|---|---|---|
| Phase A | Ethylhexyl Stearate; Phenoxyethanol; Polyglyceryl-4 Laurate; Sorbitan Laurate; Dilauryl Citrate | 4,0% |
| | Cetyl Ricinoleate | 0,8% |

(fortgesetzt)

| Phase B | | Water | ad 100,0% |
|---|---|---|---|
| | | Glycerin | 1,5% |
| Phase C | | Produkt Beispiel 3 | 1,0% |
| Phase D | | Phenoxyethanol | 0,2% |
| | | Perfume | q.s. |
| | | Preservative | q.s. |

Tab. 42: Micellares Wasser

| Water | ad 100,0% |
|---|---|
| Produkt Beispiel 2 | 4,0% |
| Glycerin | 1,5% |
| Disodium Cocoamphodiacetate | 0,5% |
| Disodium EDTA | 0,2% |
| Polyaminopropyl Biguanide | 0,2% |
| Citric Acid, 30% | ad pH 5,5 |

Tab. 43: Micellar Solution Cleanser

| Water | ad 100,0% |
|---|---|
| Butylene Glycol | 5,0% |
| Coco-Glucoside | 2,0% |
| Produkt Beispiel 2 | 2,5% |
| Glycerin | 1,0% |
| Allantoin | 0,1% |
| Curcuma Longa (Turmeric) Root Extract | 0,2% |
| Perfume | 0,2% |
| Citric Acid, 30% | ad pH 5,5 |

Tab. 44: Cleansing Water

| Water | ad 100,0% |
|---|---|
| Produkt Beispiel 1 | 2,5% |
| Phenoxyethanol; Ethylhexylglycerin | 0,9% |
| Perfume Pink Grapefruit | 0,2% |
| Glycerin | 0,5% |
| Disodium EDTA | 0,2% |
| Citric Acid, 30% | ad pH 5,5 |

Tab. 45: Micellares Wasser

| Water | ad 100,0% |
|---|---|
| Produkt Beispiel 2 | 3,0% |
| Glycerin | 1,0% |
| Capryl/Capramidopropyl Betaine | 1,5% |
| Disodium EDTA | 0,2% |
| Perfume Pink Grapefruit | 0,2% |
| Citric Acid, 30% | ad pH 5,5 |

Tab. 46: Weitere Formulierungsbeispiele

| | 46a | 46b | 46c | 46d | 46e | 46f | 46g | 46h | 46i | 46j |
|---|---|---|---|---|---|---|---|---|---|---|
| **Wasser** | ad 100% | | | | | | | | | |
| **Produkt Beispiel 1** | 2,5% | 3,5% | 3,5% | 1,0% | 3,0% | 2,5% | 5,0% | 3,0% | 3,5% | 3,5% |
| **Sodium Laureth Sulfate** | 9,0% | 8,0% | 9,0% | - | - | - | - | - | - | - |
| **Sodium Lauryl Sulfate** | - | - | - | 6,0% | - | - | - | - | 3,5% | - |
| **Cocamidopropyl Betaine** | - | 2,0% | 3,0% | 5,0% | 5,0% | 6,0% | - | - | 2,0% | 7,5% |
| **Sodium Cocoamphoacetate** | 3,0% | - | - | 1,5% | 4,5% | - | 3,5% | - | 3,5% | - |
| **Lauryl Glucoside** | - | - | - | - | 3,5% | 5,0% | 3,0% | 7,0% | - | - |
| **Coco-Glucoside** | - | 1,5% | - | - | 1,5% | 1,0% | 5,0% | 2,5% | 2,0% | - |
| **Sodium Cocoyl Glutamate** | - | - | - | - | - | 1,0% | 1,7% | 5,0% | 0,5% | - |
| **Stearic Acid** | - | - | 1,0% | - | - | - | - | 0,1% | 0,1% | 3,5% |
| **Sucrose Cocoate** | 0,5% | 1,0% | 1,0% | 1,2% | 0,3% | 0,2% | - | 1,0% | 1,0% | 1,0% |
| **Glycerin** | 0,5% | 0,3% | 0,5% | - | 0,3% | 0,5% | 1,5% | 1,0% | 0,5% | 1,0% |
| **PEG-7 Glyceryl Cocoate** | 0,3% | 0,2% | - | - | - | - | - | - | 0,5% | 0,5% |
| **Trideceth-9** | 0,2% | 0,2% | - | - | 0,2% | - | - | - | - | - |
| **Polysorbate 20** | - | 0,5% | 0,5% | - | - | - | - | - | 0,3% | 0,2% |
| **PEG-40 Hydrogenated Castor Oil** | - | 0,3% | 0,3% | - | 0,5% | - | - | - | 1,0% | - |
| **PEG-6 Caprylic/Capric Glycerides** | - | - | - | - | 0,3% | - | - | - | - | 0,2% |
| **Polyglyceryl-4 Caprate** | - | - | - | 2,0% | - | 0,5% | - | - | - | 0,5% |
| **Polyquaternium-10** | - | 0,2% | - | 0,1% | - | - | - | 0,2% | 0,2% | - |
| **Hydroxypropyl Guar Hydroxypropyltrimonium Chloride** | 0,2% | - | 0,3% | 0,2% | 0,2% | 0,3% | 0,2% | 0,1% | - | - |
| **Silicone Quaternium-22** | - | - | 0,3% | - | 0,3% | - | - | - | - | - |
| **Dimethicone** | - | 0,3% | - | - | - | - | - | - | 0,1% | - |
| **Amodimethicone** | 0,1% | 0,1% | 0,1% | 0,1% | 0,1% | - | - | - | 0,5% | - |
| **Argania Spinosa Oil** | - | - | 0,1% | 0,1% | 0,1% | - | 0,2% | - | - | - |
| **Glycol Distearate** | - | 0,5% | - | - | 0,5% | - | 0,3% | - | 0,5% | 0,5% |

(fortgesetzt)

| | 46a | 46b | 46c | 46d | 46e | 46f | 46g | 46h | 46i | 46j |
|---|---|---|---|---|---|---|---|---|---|---|
| **Isostearamide MIPA; Glyceryl Laurate** | 1,0% | - | - | 1,5% | - | - | 0,3% | - | 1,0% | 0,5% |
| **Sodium Chloride** | 0,5% | 1,8% | 2,0% | 0,5% | 1,5% | 1,8% | 0,2% | 1,0% | - | 0,5% |
| **PEG-120 Methyl Glucose Dioleate** | 0,3% | 2,5% | 1,0% | - | 1,2% | - | - | - | 0,5% | - |
| **Xanthan Gum** | - | - 0,5% | 0,5% | 0,6% | - 0,7% | 0,7% | 2,0% | 1,0% | - | - |
| **Cellulose** | - | - | - | 0,1% | - | 0,1% | 0,1% | 0,2% | 0,1% | - |
| **Zinc Pyrithione** | - | 0,1% | - | - | - | - | - | - | 0,1% | - |
| **Benzophenone-4** | - | 0,1% | 0,1% | 0,1% | 0,1% | - | 0,1% | - | 0,1% | - |
| **Tetrasodium EDTA** | 0,1% | 0,1% | | 0,1% | 0,1% | - | 0,1% | - | 0,1% | - |
| **Caffeine** | - | 0,1% | 0,1% | - | - | 0,1% | - | - | 0,1% | - |
| **Coumarin** | - | 0,1% | 0,1% | - 0,1% | 0,1% | 0,1% | 0,2% | 0,1% | 0,1% | - |
| **Panthenol** | 0,1% | 0,1% | 0,1% | 0,1% | 0,1% | 0,1% | - 0,1% | 0,1% | 0,1% | 0,1% |
| **Isopropyl Myristate** | - | - | - | 0,3% | - | 0,1% | - | - | - | 0,2% |
| **Linalool** | - | - | 0,1% | 0,1% | - | 0,1% | 0,1% | - | 0,1% | - |
| **Citrus Limon (Lemon) Peel Oil** | 0,1% | - | 0,1% | - | - | 0,3% | - | 0,2% | 0,1% | 0,2% |
| **Orange Oil** | - | 0,2% | - | - | 0,2% | - | - | 0,2% | - | - |
| **Limonene** | - | 0,1% | - | - | - | - | 0,1% | - | 0,1% | - |
| **Citric Acid** | ad pH 5,5 | | | | | | | | | |
| **Perfumes, Dyes, Preservatives** | q.s. | | | | | | | | | |

Tabelle 47: Weitere Formulierungsbeispiele

| | 47a | 47b | 47c | 47d | 47e | 47f | 47g | 47h | 47i | 47j |
|---|---|---|---|---|---|---|---|---|---|---|
| **Wasser** | ad 100% | | | | | | | | | |
| **Produkt Beispiel 2** | 7,0% | 3,0% | 2,0% | 4,0% | 4,0% | 2,5% | 4,0% | 3,5% | 3,0% | 3,0% |
| **Sodium Lauryl Sulfate** | - 8,0% | 8,0% | 9,5% | - | - | - | - | 3,5% | - | - |
| **Coco-Betaine** | - 5,0% | 5,0% | - 5,5% | 5,5% | - | - | - | 3,0% | - | - |
| **Cocamidopropyl Betaine** | - | - 3,0% | 3,0% | - | 5,0% | - | - | - | 3,0% | 2,0% |
| **Sodium Cocoamphoacetate** | - | - 2,5% | 2,5% | 3,0% | - | 5,0% | - | 3,0% | 4,0% | - |
| **Disodium Lauryl Sulfosuccinate** | - | - 1,0% | 1,0% | - | - | - | - | 1,2% | - | - |
| **Coco-Glucoside** | - | - | - 3,0% | 3,0% | 5,0% | 4,0% | 5,0% | 1,0% | - | 2,0% |
| **Sodium Cocoyl Glutamate** | - | - | - 2,5% | 2,5% | - | 3,0% | 4,5% | 0,5% | 2,5% | 0,3% |
| **Sodium Cocoyl Glycinate** | - | - | - | - | 5,0% | - | 3,5% | - | 2,0% | 7,0% |
| **Sodium Lauroyl Methyl Isethionate** | - | - | - | 1,0% | - | 1,5% | - | 1,0% | 0,5% | 0,5% |
| **Stearic Acid** | - | - 0,2% | 0,2% | - | - | - | - | 0,1% | - | 0,5% |
| **Sucrose Cocoate** | 0,5% | 0,4% | - | 1,0% | - | 0,2% | 0,2% | 0,3% | 1,0% | 0,3% |
| **Glycerin** | 1,5% | 0,3% | 0,5% | 0,5% | 0,8% | 0,5% | 1,0% | 0,5% | 0,5% | 1,0% |
| **PEG-40 Hydrogenated Castor Oil** | - 1,0% | 1,0% | - | - | - | - | - | 0,3% | - | - |
| **Polyglyceryl-4 Caprate** | 1,0% | - | - 0,5% | 0,5% | | 2,5% | - | - | 0,9% | - |
| **Polyquaternium-11** | - | 0,2% | - | - | 0,1% | - | - | 0,2% | - | 0,3% |
| **Guar Hydroxypropyltrimonium Chloride** | - | - 0,3% | 0,3% | 0,2% | 0,2% | 0,3% | 0,2% | 0,1% | 0,2% | - |
| **Dimethicone** | - 0,3% | 0,3% | - | - | - | - | - | 0,2% | - | - |
| **Aminopropyl Dimethicone** | - 0,3% | 0,3% | 0,5% | - | - | - | - | 0,3% | - | - |
| **Helianthus Annuus Seed Oil** | 0,1% | - | 0,1% | - | - 0,1% | 0,1% | - | 0,2% | - | - |
| **Dicaprylylether** | 0,5% | 0,3% | - | - | - | 0,2% | 0,3% | 0,5% | - | - |
| **Sodium Hydroxypropyl Starch Phosphate** | 0,2% | - | - | - | - | 0,5% | - | - | - | 0,5% |
| **Palmitamidopropyltrimonium Chloride** | - | - | 0,5% | - | - | - | - | 0,5% | 0,4% | - |
| **Bis-(Isostearoyl/Oleoyl Isopropyl) Dimonium Methosulfate** | 0,5% | 0,3% | - | - | - | - | 0,5% | - | - | - |

(fortgesetzt)

| | 47a | 47b | 47c | 47d | 47e | 47f | 47g | 47h | 47i | 47j |
|---|---|---|---|---|---|---|---|---|---|---|
| Glycol Distearate | 0,2% | 0,1% | 0,2% | - | 0,4% | - | - | - 0,5% | 0,5% | 0,2% |
| PEG-3 Distearate | - 0,5% | 0,5% | - | - | - | - | - | 0,5% | - | - |
| Acrylates / C10-30 Alkyl Acrylate Crosspolymer | 0,5% | - 0,4% | 0,4% | - | 0,5% | - | - | - | 0,5% | - |
| Sodium Hydroxide, 25% | 0,6% | - | 0,6% | - | 0,8% | - | - | - | 0,7% | - |
| Cocamide MEA | - | 0,8% | 1,0% | 1,0% | - | 0,4% | 0,6% | 1,0% | - | 0,3% |
| Sodium Chloride | 0,3% | 0,8% | 0,5% | - | 0,5% | 0,5% | - | 1,0% | 0,5% | 1,0% |
| Propylene Glycol; PEG-55 Propylene Glycol Oleate | - | 2,5% | - | - | - | - | - | 0,8% | - | - |
| Xanthan Gum | 0,2% | - | 0,2% | 1,2% | 0,5% | - | 1,5% | 0,2% | 1,0% | 0,9% |
| Algin | - | - | - | - | 0,7% | 1,2% | - | - | 0,5% | - |
| Benzophenone-4 | - | 0,1% | 0,2% | - | - | - | - | 0,2% | - | 0,1% |
| Menthol | 0,1% | - | 0,1% | - | - | - | - | 0,1% | 0,1% | 0,1% |
| Caffeine | - | 0,1% | 0,1% | 0,1% | 0,1% | - | - | 0,1% | - | 0,1% |
| Benzyl Alcohol | 0,1% | - | - | - | 0,1% | 0,1% | - | 0,1% | - | - |
| Hydrolyzed Wheat Protein | - | - 0,1% | 0,1% | 0,1% | 0,1% | 0,2% | 0,2% | 0,1% | - | - |
| Octopirox | 0,2% | - | - | - | - | 0,1% | - | - | - | - |
| Salicylic Acid | 0,1% | - | - | - | 0,1% | 0,1% | - | - | 0,1% | - |
| 1,2-Hexanediol | - | - | - | 0,2% | - | 0,5% | 0,5% | 0,2% | - | - |
| Isopropyl Myristate | - | 0,3% | - | 0,2% | - | - | - | 0,5% | 0,3% | - |
| Linalool | - | 0,1% | 0,1% | 0,1% | - | 0,1% | 0,1% | 0,1% | 0,1% | - |
| Citrus Limon (Lemon) Peel Oil | 0,1% | - | 0,1% | 0,3% | - | - 0,2% | - | 0,1% | 0,1% | 0,2% |
| Orange Oil | - | 0,2% | - | 0,1% | 0,5% | - | - | - | - | - |
| Panthenol | 0,1% | 0,1% | - | 0,1% | 0,1% | 0,1% | 0,1% | 0,1% | 0,1% | 0,1% |
| Citric Acid | ad pH 5,2 | | | | | | | | | |
| Perfumes, Dyes, Preservatives | q.s. | | | | | | | | | |

**Tabelle 48: Liste der eingesetzten Rohstoffe**

| INCI | Handelsname, Firma |
|---|---|
| 1,2-Hexanediol | Hydrolite-6 841129, Symrise |
| Acrylates/Beheneth-25 Methacrylate Copolymer | Novethix L-10 Polymer, Lubrizol |
| Acrylates / C10-30 Alkyl Acrylate Crosspolymer | TEGO Carbomer 841 SER, Evonik Nutrition & Care GmbH, 100% |
| Algin | Hydagen 558 P, BASF |
| Allantoin | Allantoin, DSM Nutritional Products, Inc. |
| Aloe Barbadensis Leaf Juice | Aloe-Con UP 40, Florida Food Products Inc. |
| alpha-Isomethyl Ionone | alpha-Isomethylionone, Chemos GmbH |
| Alumina | Aeroxide Alu C, Evonik Nutrition & Care GmbH |
| Aluminum Chlorohydrate | Locron L, Clariant |
| Ammonium Lauryl Sulfate | Empicol AL 70, Albright & Wilson UK Limited |
| Aminopropyl Dimethicone | ABIL Soft AF 200, Evonik Nutrition & Care GmbH |
| Amodimethicone | DC 949, Dow Corning, 100% |
| Argania Spinosa Oil (Argania Spinosa Kernel Oil) | Argan Oil, DSM Nutritional Products Ltd. |
| Benzophenone-4 | Uvinul MS 40, BASF Corporation |
| Benzoic Acid | OriStar BZA, Orient Stars LLC |
| Benzyl Alcohol | Microcare BNA, THOR PERSONAL CARE SAS |
| Benzyl Salicylate | Seridefrizz Intense, Cheemyunion Quimica Ltda. |
| Bis-(Isostearoyl/Oleoyl Isopropyl) Dimonium Methosulfate | VARISOFT EQ 100, Evonik Nutrition & Care GmbH, 100% |
| Butylene Glycol | Butylene Glycol, Oxea Corparation |
| Butyrospermum Parkii Butter Extract | Cosmosil 600, International Cosmetic Science Centre |
| Caffeine | Caffeine, Merck KGaA/EMD Chemicals, Inc. |
| Camellia Oleifera Seed Oil | Camellia Sasanqua Oil, Ikeda Corporation |
| Caprylyl Glycol | Sensiva SC 10, Schülke& Mayr GmbH |
| Caprylyl/Capryl Glucoside | |
| Capryl/Capramidopropyl Betaine | TEGO Betaine 810, Evonik Nutrition & Care GmbH, 38% |
| Caprylic/Capric Triglyceride | TEGOSOFT CT, Evonik Nutrition & Care GmbH, 100% |
| Carbomer | TEGO Carbomer 140, Evonik Nutrition & Care GmbH, 100% |
| Cellulose | Arbocel A300, J. Rettenmaier & Söhne |
| Cetyl Ricinoleate | TEGOSOFT CR, Evonik Nutrition & Care GmbH, 100% |
| Cetrimonium Bromide | Rhodaquat M-242B/99, Rhodia |
| Chamomilla Recutita (Matricaria) Extract | Recentia CR, AkzoNobel Global Personal Care |

(fortgesetzt)

| INCI | Handelsname, Firma |
| --- | --- |
| Citral | Citral FF, Symrise AG |
| Citric Acid | Citric Acid USP Granular, DSM Nutritional Products, Inc. |
| Citrus Aurantifolia (Lime) Oil | AEC Lime Oil, A & E Connock, Perfumery & Cosmetics Ltd. |
| Cocamide DEA | REWOMID DC 212 S, Evonik Nutrition & Care GmbH, 100% |
| Cocamide MEA | REWOMID D 212, Evonik Nutrition & Care GmbH, 100% |
| Cocamidopropyl Betaine | TEGO Betain F 50, Evonik Nutrition & Care GmbH, 38% |
| Coco-Glucoside | Plantacare 818 UP, BASF Cognis, 51% |
| Coco-Betaine | Dehyton AB 30, BASF Cognis, 31% |
| Coumarin | Rhodiascent extra pure, Rhodia Organics |
| Curcuma Longa (Turmeric) Root Extract | TEGO Turmerone, Evonik Nutrition & Care GmbH |
| Decyl Glucoside | Plantacare 2000 UP, BASF Cognis |
| Decyl Oleate | TEGOSOFT DO, Evonik Nutrition & Care GmbH, 100% |
| Dicaprylyl Ether | Cetiol OE, BASF Cognis |
| Dehydroacetic Acid | Unisept DHA (Universal Preserv-A-Chem, Inc.) |
| Dimethicone | DC 200 Fluid 100 cSt, Dow Corning, 100% |
| Disodium Cocoamphodiacetate | Rewoteric AM 2 C NM, Evonik Nutrition & Care GmbH |
| Disodium Cocoyl Glutamate | Planatpon ACG LC, BASF Cognis |
| Disodium EDTA | Dissolvine NA-2-P, AkzoNobel Global Personal Care |
| Disodium Lauryl Sulfosuccinate | REWOPOL SB F 12 P, Evonik Nutrition & Care GmbH, 95% |
| Distearyl Ether | Cosmacol SE, Sasol Germany GmbH |
| Ethylhexylglycerin | Sensiva SC 50, Schuelke & Mayr GmbH |
| Ethylhexyl Stearate; Phenoxyethanol; Polyglyceryl-4 Laurate; Sorbitan Laurate; Dilauryl Citrate | TEGO Wipe Flex, Evonik Nutrition & Care GmbH |
| Geraniol | Nerol 800, International Flavors & Fragrances Inc. |
| Glucose | Organic Biovert Substrate, Lonza |
| Glycerin | Glycerol EP, vegetable, Spiga Nord, 99,7% |
| Glyceryl Caprylate | Dermosoft GMCY, Dr. Straetmans |
| Glyceryl Isostearate | Peceol Isostearique, Gattefosse |
| Glyceryl Oleate | TEGIN O V, Evonik Nutrition & Care GmbH |
| Glycine Soja (Soybean) Oil | Cropure Soybean, Croda Europe, Ltd. |

(fortgesetzt)

| INCI | Handelsname, Firma |
|---|---|
| Glycol Distearate | TEGIN G 1100 Pellets, Evonik Nutrition & Care GmbH, 100% |
| Guar Hydroxypropyltrimonium Chloride | Cosmedia Guar C 261, BASF Personal Care and Nutrition Gmbh / Jaguar C-17, Rhodia Inc. und andere |
| Helianthus Annuus (Sunflower) Seed Oil | AEC Sunflower Oil, A & E Connock, Perfumery & Cosmetics Ltd. |
| Hydrolyzed Silk | Crosilk 10000, Croda Inc. |
| Hydrolyzed Wheat Protein | Gluadin WLM, BASF Cognis |
| Hydrolyzed Wheat Starch | Cropeptide W, Croda, Inc. |
| Hydroxyethyl Ethylcellulose | Structure Cel 4400 E, AkzoNobel Global Personal Care |
| Hydroxypropyl Guar Hydroxypropyltrimonium Chloride | Jaguar C-162, Rhodia, 100% |
| Hydroxypropyl Methylcellulose | TEGOCEL HPM 50, Evonik Nutrition & Care GmbH, 100% |
| Isoceteth-20 | TEGO Alkanol IC 20, Evonik Nutrition & Care GmbH, 100% |
| Isopropyl Myristate | TEGOSOFT M, Evonik Nutrition & Care GmbH, 100% |
| Isopropyl Palmitate | TEGOSOFT P, Evonik Nutrition & Care GmbH, 100% |
| Isostearamide MIPA; Glyceryl Laurate | ANTIL SPA 80, Evonik Nutrition & Care GmbH, 100% |
| Lactic Acid | AEC Lactic Acid, A & E Connock, Perfumery & Cosmetics Ltd. |
| Lactis Proteinum | AEC Whey Protein, A & E Connock, Perfumery & Cosmetics Ltd. |
| Laureth-5 Carboxylic Acid | Marlowet 1072, Sasol Germany GmbH - Marl |
| Lauric Acid | Prifrac 2920, Croda Europe, Ltd. |
| Lauryl Glucoside | Plantacare 1200 UP, BASF Cognis, 50% |
| Lavandula Angustifolia (Lavender) Oil | AEC Lavender Oil, A&E Connock Ltd. |
| Lecithin | AEC Lecithin Powder, A & E Connock, Perfumery & Cosmetics Ltd. |
| Limonene | Dipentene No. 122, Hercules Inc. |
| Citrus Limon (Lemon) Peel Oil | Fragrance Resources |
| Cymbopogon Schoenanthus (Lemongrass) Oil | AEC Lemongrass Oil, A&E Connock Ltd. |
| Linalool | Lipofresh, Lipo Chemicals, Inc. |
| Magnesium Chloride | OriStar MCL, Orient Stars LLC |
| Maltodextrin | Farmal MD 10, Corn Products International |
| Malva Sylvestris (Mallow) Leaf Extract | Herbasec Mallow Leaves, Cosmetochem International AG |
| Mangifera Indica (Mango) Fruit Extract | Mango Extract, Draco Natural Products |

(fortgesetzt)

| INCI | Handelsname, Firma |
|------|--------------------|
| Menthol | OriStar MC, Orient Stars LLC |
| Methylisothiazolinone | Microcare MT, Thor Specialties, Inc. |
| Mentha Piperita (Peppermint) Oil | AEC Peppermint Oil, A&E Connock Ltd. |
| MIPA-Laureth Sulfate | Zetesol 2056, Zschimmer & Schwarz GmbH |
| Octopirox | Octopirox, Clariant Intl. Ltd. |
| Olea Europaea (Olive) Fruit Oil | Cropure Olive, Croda Europe, Ltd. |
| Orange Oil | Fragrance Resources |
| Palmitamidopropyltrimonium Chloride | VARISOFT PATC, Evonik Nutrition & Care GmbH, 60% |
| Panthenol | D-Panthenol USP, BASF, 100% |
| PEG-120 Methyl Glucose Dioleate | ANTIL 120 Plus, Evonik Nutrition & Care GmbH, 100% |
| PEG-150 Distearate | REWOPAL PEG 6000 DS A, Evonik Nutrition & Care GmbH, 100% |
| PEG-18 Castor Oil Dioleate | Marlowet CG, Sasol Germany GmbH |
| PEG-18 Glyceryl Oleate/Cocoate | ANTIL 171, Evonik Nutrition & Care GmbH, 100% |
| PEG-200 Hydrogenated Glyceryl Palmate; PEG-7 Glyceryl Cocoate | REWODERM LI S 80, Evonik Nutrition & Care GmbH, 100% |
| PEG-3 Distearate | TEGIN D 1102, Evonik Nutrition & Care GmbH, 100%; Cutina TS, BASF Cognis, 100% |
| PEG-40 Hydrogenated Castor Oil | TAGAT CH 40, Evonik Nutrition & Care GmbH, 100% |
| PEG-6 Caprylic/Capric Glycerides | TEGOSOFT GMC-6, Evonik Nutrition & Care GmbH, 100% |
| PEG-7 Glyceryl Cocoate | TEGOSOFT GC, Evonik Nutrition & Care GmbH, 100% |
| Perfume Pink Grapefruit | Fragrance Resources |
| Perfume Spicy Herbs | Fragrance Resources |
| Persea Gratissima (Avocado) Oil | Cropure Avocado, Croda Europe, Ltd. |
| Petrolatum | Merkur 115, Sasol Wax GmbH |
| Phenethyl Alcohol | Etaphen, Vevy Europe SpA |
| Phenoxyethanol | S&M Phenoxyethanol, Schülke & Mayr GmbH |
| Phenoxyethanol; Ethylhexylglycerin | Euxyl PE 9010, Schülke & Mayr GmbH |
| Phenoxyethanol; Methylparaben; Ethylparaben; Butylparaben; Propylparaben; Isobutylparaben | Euxyl K 300, Schuelke & Mayr GmbH |
| Polyaminopropyl Biguanide | Microcare MBG, Thor |
| Polyglyceryl-3 Palmitate | Dermofeel PP, Dr. Straetmans |
| Polyglyceryl-4 Caprate | TEGOSOFT PC 41, Evonik Nutrition & Care GmbH, 100% |

(fortgesetzt)

| INCI | Handelsname, Firma |
|---|---|
| Polyglyceryl-6 Caprylate; Polyglyceryl-3 Cocoate; Polyglyceryl-4 Caprate; Polyglyceryl-6 Ricinoleate | TEGO Solve 61, Evonik Nutrition & Care GmbH, |
| Polyquaternium-10 | Polymer JR 400, Amerchol, 100% |
| Polyquaternium-11 | Dehyquart CC 11, BASF Personal Care and Nutrition Gmbh / Luviquat PQ 11 PN, BASF Corporation |
| Polyquaternium-7 | Merquat 550, Nalco, 100% |
| Polysorbate 20 | TEGO SML 20, Evonik Nutrition & Care GmbH, 100% |
| Potassium Sorbate | Euxyl K 712, Schülke & Mayr GmbH |
| Propylene Glycol | Euxyl K 320, Schülke & Mayr GmbH |
| Propylene Glycol; PEG-55 Propylene Glycol Oleate | ANTIL 141 Liquid, Evonik Nutrition & Care GmbH |
| Prunus Amygdalus Dulcis (Sweet Almond) Oil | Cropure Almond, Croda Europe, Ltd. |
| Prunus Cerasus (Bitter Cherry) Fruit Extract | Prunus Cerasus Fruit, Kirschen Extract, Botanica GmbH |
| Ricinus Communis Seed Oil | Lipovol CO, Lipo Chemicals |
| Rosemary Oil | Fragrance Resources |
| Salvia Officinalis (Sage) Oil | AEC Sage Oil, A&E Connock Ltd. |
| Salicylic Acid | OriStar SCA, Orient Stars LLC |
| Silica | Aerosil 130, Evonik Degussa GmbH |
| Silicone Quaternium-22 | ABIL T Quat 60, Evonik Nutrition & Care GmbH, 65% |
| Silicone Quaternium-22; Polyglycerin-3 Caprate; Dipropylene Glycol; Cocamidopropyl Betaine | ABIL ME 45, Evonik Nutrition & Care GmbH, 30% |
| Simmondsia Chinensis (Jojoba) Seed Oil | AEC Jojoba Oil Refined, A & E Connock, Perfumery & Cosmetics Ltd. |
| Sodium Benzoate | Euxyl K 712, Schülke & Mayr GmbH |
| Sodium Cetearyl Sulfate | Lanette E, BASF Personal Care and Nutrition Gmbh |
| Sodium Cocoamphoacetate | REWOTERIC AM C, Evonik Nutrition & Care GmbH, 32% |
| Sodium Cocoamphopropionate | REWOTERIC AM KSF 40, Evonik Nutrition & Care GmbH, 40% |
| Sodium Coco-Sulfate | Texapon HC G, BASF |
| Sodium Cocoyl Glutamate | Plantapon ACG HC, BASF Cognis |
| Sodium Cocoyl Glycinate | Hostapon SG, Clariant; Amilite GCS-11, Ajinomoto |
| Sodium/Disodium Cocoyl Glutamate | PERLASTAN SC 25 NKW, Schill&Seilacher, 25%-ig, |
| Sodium Hydroxide | Unichem SOHYD, Universal Preserv-A-Chem, Inc. |

(fortgesetzt)

| INCI | Handelsname, Firma |
|---|---|
| Sodium Hydroxypropyl Starch Phosphate | Pure-Gel, Grain Processing Corporation |
| Sodium Isethionate | Hostapon SI, Company Clariant International Ltd, |
| Sodium Lactate | Sodium Lactate Solution About 50%, Merck KGaA/EMD Chemicals, Inc. |
| Sodium Lactate; Sodium PCA; Glycine; Fructose; Urea; Niacinamide; Inositol; Sodium Benzoate; Lactic Acid | LACTIL, Evonik Nutrition & Care GmbH |
| Sodium Laureth Sulfate | Texapon NSO, BASF Cognis, 28% |
| Sodium Lauroamphoacetate | ColaTeric SLAA, Colonial Chemical Inc |
| Sodium Lauroyl Isethionate | Yongan SLI , Huanggang Yongan Pharmaceutical Co.,Ltd |
| Sodium Lauroyl Methyl Isethionate | Iselux, Innospec Active Chemicals |
| Sodium Lauryl Sulfate | Texapon LS 35, BASF Cognis, 30% |
| Sodium Trideceth Sulfate | Rhodapex EST-30, Rhodia |
| Sorbitan Caprylate | Sorbon S-10, Toho Chemical Industry Co., Ltd. |
| Stearic Acid | Pristerene 4922, Croda Europe, Ltd. |
| Stearyl Alcohol | TEGO Alkanol 18, Evonik Nutrition & Care GmbH |
| Styrene/Acrylates Copolymer | Acudyne HS, The Dow Chemical Company |
| Sucrose Cocoate | TEGOSOFT LSE 65 K, Evonik Nutrition & Care GmbH, 100% |
| Tetrasodium EDTA | Versene 100, The Dow Chemical Company |
| Tocopherol | Euxyl K 700, Schülke & Mayr GmbH |
| Trideceth-9 | Marlipal O 13/90, Sasol Germany GmbH - Marl |
| Triticum Vulgare Germ Oil | Cropure Wheatgerm, Croda Europe, Ltd. |
| Xanthan Gum | Keltrol CG-SFT, CP Kelco, 100% |
| Zinc Pyrithione | Microcare ZP, THOR PERSONAL CARE SAS |

**Patentansprüche**

1. Polyglycerinpartialester erhältlich durch Veresterung eines Polyglycerins mit einer Carbonsäuremischung umfassend:

   a) mindestens eine kurzkettige Dicarbonsäure aufweisend 2 bis 12, bevorzugt 3 bis 8, besonders bevorzugt 4, Kohlenstoffatome, und
   b) mindestens eine gesättigte Fettsäure aufweisend 6 bis 14, bevorzugt 8 bis 10, Kohlenstoffatome,

   **dadurch gekennzeichnet, dass** das molare Verhältnis von Polyglycerin zu Dicarbonsäure zu gesättigter Fettsäure in einem Verhältnis von 3,1 : 1,0 : 0,5 bis 14 : 1,0 : 6,0, bevorzugt von 3,3 : 1,0 : 0,7 bis 7,0 : 1,0 : 3,0.

2. Polyglycerinpartialester gemäß Anspruch 1, **dadurch gekennzeichnet, dass** das Polyglycerin einen mittleren Kondensationsgrad $N$, gemessen wie in der Beschreibung ausgeführt, von 1,5 bis 9, bevorzugt 2 bis 7, besonders bevorzugt 2,5 bis 5, aufweist.

3. Polyglycerinpartialester gemäß Anspruch 1 oder 2, **dadurch gekennzeichnet, dass** das eingesetzte Polyglycerin eine Hydroxylzahl, gemessen wie in der Beschreibung ausgeführt, von 1500 bis 900, bevorzugt 1350 bis 940, besonders bevorzugt 1245 bis 1010 mg KOH / g aufweist.

4. Polyglycerinpartialester gemäß Anspruch 1 oder 2, **dadurch gekennzeichnet, dass** die kurzkettige Dicarbonsäure ausgewählt ist aus aliphatischen, linearen Dicarbonsäuren, insbesondere Oxalsäure, Malonsäure, Bernsteinsäure, Glutarsäure, Adipinsäure, Pimelinsäure, Korksäure, Azelainsäure, Sebacinsäure, Dodecandisäure, wobei Bernsteinsäure besonders bevorzugt ist.

5. Polyglycerinpartialester gemäß mindestens einem der vorherigen Ansprüche, **dadurch gekennzeichnet, dass** die gesättigte Fettsäure ausgewählt ist aus unverzweigten, unsubstituierten Fettsäuren.

6. Polyglycerinpartialester gemäß mindestens einem der vorherigen Ansprüche, **dadurch gekennzeichnet, dass** in der Veresterung das molare Verhältnis von a) zu b)
von 1,0 : 0,7 bis 1,0 : 3,0, bevorzugt
von 1,0 : 0,8 bis 1,0 : 2,0, besonders bevorzugt
von 1,0 : 1,0 bis 1,0 : 1,6.
beträgt.

7. Polyglycerinpartialester gemäß mindestens einem der vorherigen Ansprüche, **dadurch gekennzeichnet, dass** er bei 1 bar einen Trübungspunkt, gemessen wie in der Beschreibung ausgeführt, von 45 bis 75 °C, bevorzugt von 50 bis 65 °C, aufweist.

8. Polyglycerinpartialester gemäß mindestens einem der vorherigen Ansprüche, **dadurch gekennzeichnet, dass** er einen HLB-Wert, gemessen wie in der Beschreibung ausgeführt, von 13 bis 17 aufweist.

9. Polyglycerinpartialester gemäß mindestens einem der vorherigen Ansprüche, **dadurch gekennzeichnet, dass** er eine Oberflächenspannung, gemessen wie in der Beschreibung ausgeführt, von weniger als 28 mN/m in 1,0%iger wässriger Lösung bei 20 °C aufweist.

10. Polyglycerinpartialester gemäß mindestens einem der vorherigen Ansprüche, **dadurch gekennzeichnet, dass** er eine Verseifungszahl, gemessen wie in der Beschreibung ausgeführt, von 70 bis 199 mg KOH / g, bevorzugt von 95 bis 190, besonders bevorzugt von 120 bis 180 mg KOH / g, aufweist.

11. Verwendung mindestens eines Polyglycerinpartialesters gemäß mindestens einem der Ansprüche 1 bis 10 als Solubilisator, insbesondere in kosmetischen oder pharmazeutischen Zubereitungen.

**Claims**

1. Polyglycerol partial ester obtainable by esterification of a polyglycerol with a carboxylic acid mixture comprising:

   a) at least one short-chain dicarboxylic acid having 2 to 12, preferably 3 to 8, particularly preferably 4 carbon atoms, and
   b) at least one saturated fatty acid having 6 to 14, preferably 8 to 10 carbon atoms,

   **characterized in that** the molar ratio of polyglycerol to dicarboxylic acid to saturated fatty acid is in a ratio of from 3.1 : 1.0 : 0.5 to 14 : 1.0 : 6.0, preferably from 3.3 : 1.0 : 0.7 to 7.0 : 1.0 : 3.0.

2. Polyglycerol partial ester according to Claim 1, **characterized in that** the polyglycerol has an average degree of condensation N, measured as stated in the description, of 1.5 to 9, preferably 2 to 7, particularly preferably 2.5 to 5.

3. Polyglycerol partial ester according to Claim 1 or 2, **characterized in that** the polyglycerol used has a hydroxyl number, measured as stated in the description, of 1500 to 900, preferably 1350 to 940, particularly preferably 1245 to 1010 mg KOH / g.

4. Polyglycerol partial ester according to Claim 1 or 2, **characterized in that** the short-chain dicarboxylic acid is selected from aliphatic, linear dicarboxylic acids, in particular oxalic acid, malonic acid, succinic acid, glutaric acid, adipic acid, pimelic acid, suberic acid, azelaic acid, sebacic acid, dodecanedioic acid, wherein succinic acid is particularly preferred.

5. Polyglycerol partial ester according to at least one of the preceding claims, **characterized in that** the saturated fatty acid is selected from unbranched, unsubstituted fatty acids.

6. Polyglycerol partial ester according to at least one of the preceding claims, **characterized in that** in the esterification the molar ratio of a) to b) is

from 1.0 : 0.7 to 1.0 : 3.0, preferably
from 1.0 : 0.8 to 1.0 : 2.0, more preferably
from 1.0 : 1.0 to 1.0: 1.6.

7. Polyglycerol partial ester according to at least one of the preceding claims, **characterized in that** at 1 bar it has a turbidity point, measured as stated in the description, of 45 to 75°C, preferably 50 to 65°C.

8. Polyglycerol partial ester according to at least one of the preceding claims, **characterized in that** it has an HLB value, measured as stated in the description, of 13 to 17.

9. Polyglycerol partial ester according to at least one of the preceding claims, **characterized in that** it has a surface tension, measured as stated in the description, of less than 28 mN/m in 1.0% aqueous solution at 20°C.

10. Polyglycerol partial ester according to at least one of the preceding claims, **characterized in that** it has a saponification number, measured as stated in the description, of 70 to 199 mg KOH / g, preferably 95 to 190, particularly preferably 120 to 180 mg KOH / g.

11. Use of at least one polyglycerol partial ester according to at least one of Claims 1 to 10 as solubilizer, particularly in cosmetic or pharmaceutical preparations.

**Revendications**

1. Ester partiel de polyglycérine pouvant être obtenu par estérification d'une polyglycérine avec un mélange d'acides carboxyliques, comprenant :

a) au moins un acide dicarboxylique à chaîne courte comprenant 2 à 12, de préférence 3 à 8, de manière particulièrement préférée 4, atomes de carbone, et
b) au moins un acide gras saturé comprenant 6 à 14, de préférence 8 à 10, atomes de carbone,

**caractérisé en ce que** le rapport molaire entre la polyglycérine et l'acide dicarboxylique et l'acide gras saturé est de 3,1:1,0:0,5 à 14:1,0:6,0, de préférence de 3,3:1,0:0,7 à 7,0:1,0:3,0.

2. Ester partiel de polyglycérine selon la revendication 1, **caractérisé en ce que** la polyglycérine présente un degré de condensation moyen N, mesuré tel qu'indiqué dans la description, de 1,5 à 9, de préférence de 2 à 7, de manière particulièrement préférée de 2,5 à 5.

3. Ester partiel de polyglycérine selon la revendication 1 ou 2, **caractérisé en ce que** la polyglycérine utilisée présente un indice hydroxyle, mesuré tel qu'indiqué dans la description, de 1 500 à 900, de préférence de 1 350 à 940, de manière particulièrement préférée de 1 245 à 1 010 mg de KOH/g.

4. Ester partiel de polyglycérine selon la revendication 1 ou 2, **caractérisé en ce que** l'acide dicarboxylique à chaîne courte est choisi parmi les acides dicarboxyliques aliphatiques linéaires, notamment l'acide oxalique, l'acide malonique, l'acide succinique, l'acide glutarique, l'acide adipique, l'acide pimélique, l'acide subérique, l'acide azélaïque, l'acide sébacique, l'acide dodécanedioïque, l'acide succinique étant particulièrement préféré.

5. Ester partiel de polyglycérine selon au moins l'une quelconque des revendications précédentes, **caractérisé en ce que** l'acide gras saturé est choisi parmi les acides gras non ramifiés et non substitués.

6.  Ester partiel de polyglycérine selon au moins l'une quelconque des revendications précédentes, **caractérisé en ce que**, lors de l'estérification, le rapport molaire entre a) et b) est de 1,0:0,7 à 1,0:3,0, de préférence de 1,0:0,8 à 1,0:2,0, de manière particulièrement préférée de 1,0:1,0 à 1,0:1,6.

7.  Ester partiel de polyglycérine selon au moins l'une quelconque des revendications précédentes, **caractérisé en ce qu'**il présente à 1 bar un point de trouble, mesuré tel qu'indiqué dans la description, de 45 à 75 °C, de préférence de 50 à 65 °C.

8.  Ester partiel de polyglycérine selon au moins l'une quelconque des revendications précédentes, **caractérisé en ce qu'**il présente une valeur HLB, mesurée tel qu'indiqué dans la description, de 13 à 17.

9.  Ester partiel de polyglycérine selon au moins l'une quelconque des revendications précédentes, **caractérisé en ce qu'**il présente une tension de surface, mesurée tel qu'indiqué dans la description, inférieure à 28 mN/m dans une solution aqueuse à 1,0 % à 20 °C.

10. Ester partiel de polyglycérine selon au moins l'une quelconque des revendications précédentes, **caractérisé en ce qu'**il présente un indice de saponification, mesuré tel qu'indiqué dans la description, de 70 à 199 mg de KOH/g, de préférence de 95 à 190, de manière particulièrement préférée de 120 à 180 mg de KOH/g.

11. Utilisation d'au moins un ester partiel de polyglycérine selon au moins l'une quelconque des revendications 1 à 10 en tant que solubilisateur, notamment dans des préparations cosmétiques ou pharmaceutiques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012007754 A
- EP 0835862 A
- EP 1683781 A
- EP 1500427 A

**In der Beschreibung aufgeführte Nicht-Patentliteratur**

- **CASSEL et al.** Original synthesis of linear, branched and cyclic oligoglycerol Standards. *J. Org. Chem.,* 2001, 875-896
- Classification of surface active agents. **GRIFFIN, W. C.** J. Soc. Cosmet. Chem. HLB, 1949, vol. 1, 13-17